# EUROPEAN PATENT SPECIFICATION

(11) **EP 2 233 512 B1**
(45) Date of publication and mention of the grant of the patent: **20.03.2013**
(21) Application number: 08866590.6
(22) Date of filing: 19.12.2008
(51) Int. Cl.: C08G 73/10, C07D 209/48

(54) **POLYTHIOETHERIMIDES AND METHOD FOR PRODUCING THEREOF**
POLYTHIOETHERIMIDE UND VERFAHREN ZU IHRER HERSTELLUNG
POLYTHIOÉTHERIMIDES ET PROCÉDÉ POUR PRODUIRE CEUX-CI

(30) Priority: 19.12.2007 CN 200710300814; 14.03.2008 CN 200810060189
(43) Date of publication of application: 29.09.2010
(73) Proprietor: Ningbo Institute Of Materials Technology And Engineering, Chinese Academy Of Sciences, Zhejiang 315201 (CN); Changchun Hipolyking Co., Ltd., Hi-Tech Development Zone Changchun Jilin 130103 (CN)
(72) Inventor: FANG, Xingzhong, Ningbo Zhejiang 315201 (CN); HU, Benlin, Ningbo Zhejiang 315201 (CN); HAN, Ying, Ningbo Zhejiang 315201 (CN); MA, Ji, Ningbo Zhejiang 315201 (CN); YAN, Qing, Ningbo Zhejiang 315201 (CN); DING, Mengxian, Changchun Jilin 130022 (CN)
(74) Representative: Bird, William Edward
(86) International application number: PCT/CN2008/073592
(87) International publication number: WO 2009/082942

(56) References cited:
- JP-A- 63 301 864
- US-A- 2 443 888
- US-A- 4 092 297

## Description

### Technical Field

The invention is directed to polythioetherimides and methods for preparing the same, in particular to methods for preparing novel polythioetherimides using chlorophthalic anhydride or nitrophthalic anhydride isomers as starting materials.

### Background Art

Despite the superior comprehensive performances owned by polyimides, a typical class of thermally stable high molecular materials, resulting from rigid imide units present in their molecular chains, their low solubility and poor melt processability restrict their development and application. Polythioetherimides, obtained by incorporating soft units of ether bond into the rigid backbones of polyimides, are characterized by good solubility, low melt viscosity and melt processability in addition to excellent thermal mechanical properties. The best known product of this kind is the engineering plastics developed by GE under the name of Ultem. According to various methods disclosed in US patent applications No. 3847867, 3814860, 3850885, 3852242, 3855178, 3983093, 5830974 and so on, polythioetherimides are generally prepared via reactions between ether-bond-containing dianhydride and diamine monomers or between ether-bond-containing diamine and dianhydride monomers. Alternatively, they can be prepared via aromatic nucleophilic substitutions between disubstituted phthalic imide monomers and salts of bisphenols. The alternative methods draw extensive attention for less steps and lower cost are required. More recently, US patent application No. 6849706 discloses a novel class of isomeric copolyetherimides and methods for preparing the same.

Polythioetherimides are generally prepared via reactions between dianhydrides of thioether-bond-containing aromatic tetrabasic acids and aliphatic or aromatic diamines. Due to the incorporation of soft units of thioether bond into the rigid backbones of polyimides, such polymers are characterized by good solubility, low melt viscosity and melt processability in addition to excellent thermal mechanical properties, which lead them to be promising thermoplastic high molecular materials with high thermal resistance. Therefore, it was very early when the synthesis of diphenyl thioether type tetrabasic acid dianhydrides and corresponding polythioetherimides aroused people's interest.

US 2,443,888A discloses a product comprising a wax-like material having an acid number below 35 and a melting point of the order of 120°C, and above, which is stable at temperatures at least as high as its melting point, the said material comprising a complex, heat-reacted mixture of N-amyl-substituted tetrachlorophthalimides corresponding to the general formula 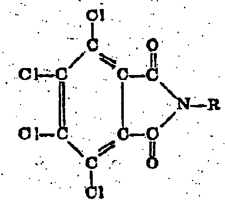 where R represents a five-membered hydrocarbon radical derived from normal and isomeric monoamyl amines, the said wax-like material having been obtained by interaction under heat between tetrachlorophthalic anhydride and a mixture of monoamines including a plurality of monoamyl amines consisting essentially of tertiary amyl amine, secondary isoamyl amine, 2-aminopentane, 3-aminopentane, active amyl amine, isoamyl amine, and normal amyl amine, the said mixture of amines having an initial; boiling point of at least 84°C, at least 95% distilling off below 100°C, and a final boiling point not higher than 110°C.

US 4,092,297 discloses a method of making polythioetherimide which comprises effecting reaction in the presence of a cresol solvent between from 0.5 to 2 moles of aromatic bis(thioetheranhydride) of the formula 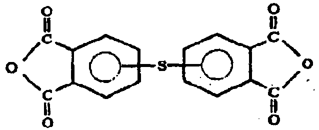 per mole of organic diamine of the formula, NH₂NRNH₂ at temperatures of from 100° to 250°C, where R is a divalent organic radical selected from the class consisting of (a) aromatic hydrocarbon radicals having from 6-20 carbon atoms and halogenated derivatives thereof, (b) alkylene radicals and cycloalkylene radicals having from 2-20 carbon atoms, C₍₂₋₈₎ alkylene terminated polydiorganosiloxane, and (c) divalent radicals included by the formula, 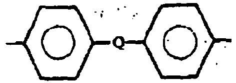 where Q is a member selected from the class consisting of -O-, -C(=O)-,-S(=O)₂-. -S-, and -CₓH₂ₓ-, and x is a whole number from 1 to 5 inclusive.

Furthermore, US 3989712, 4054584, 4499285 and 4625037 reported the synthesis of 3,3' -position diphenyl thioether dianhydride or 4,4' -position diphenyl thioether dianhydride wherein 3- or 4-nitro- (or chloro-) phthalic imide reacted with an alkali metal sulfide or hydro sulfide, such as sodium sulfide or sodium hydro sulfide, to give a corresponding intermediate, i.e. thioether bisimide, which was hydrolyzed, acidified and dehydrated to obtain the object product; CN 1081436 reported the method for preparing 3,3'-, 3,4'- or 4,4'-position diphenyl thioether tetrabasic acid and dianhydride thereof using a halogen substituted phthalic anhydride as the starting material and sulfur as the sulfurating agent; and CN1724528 reported the method for preparing 3,4'-position diphenyl thioether dianhydride using chlorophthalic anhydride as the starting material and sodium hydro sulfide as the sulfurating agent. These dianhydrides might react with diamines to give corresponding polythioetherimidcs. In addition, US 4092297 and Poly Bull 1995, 34 287-294 reported the production of polythioether-imides via reactions between 3,3'- or 4,4'-position dinitro- (or dichloro-) phthalic imides and alkali metal sulfides such as sodium sulfide. Although these polythioetherimides have superior thermal resistance and mechanical performances, they suffer from tedious preparing procedure and relatively high cost. Furthermore, their processabilities such as melt processability and solution processability need to be improved.

### Summary of the Invention

The first object of the invention is to provide a novel polythioetherimide.

The second object of the invention is to provide a novel method for preparing the polythioetherimide.

The third object of the invention is to provide an alternative method for preparing the polythioetherimide.

In the first aspect of the invention, a novel polythioetherimide of formula I is provided: 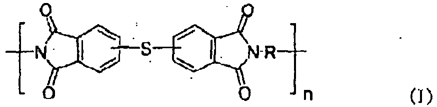 wherein thioether bond may be located at 3-position or 4-position, wherein the indicated 3-position and 4-position refer to the substitution positions of all phthalic imide rings in the polymer, wherein the organic group R is a substituted or unsubstituted aliphatic or aromatic diamine and wherein the polyetherimide has a logarithmic viscosity number of 0.13 dUg to 1.90 dL/g measured in 0.5 g/dL m-cresol at 30°C, a weight average molecular weight of 3,000 to 200,000 and a polydispersity of 1.8 to 5.4. The polythioetherimide obtained according to the invention exhibits excellent comprehensive performances, such as good thermal resistance, high flexibility, low melt viscosity, etc.. The polymer resins are suited to be processed by injection molding, extrusion molding, press molding, solution spinning and melt spinning, and therefore their promising applications in high temperature resistant engineering plastics, thin films, adhesive agents, enameled wires, foam plastics, fibers and advanced composite materials are predictable.

In the second aspect of the invention, a method for preparing a polythioetherimide is provided, wherein chlorophthalic anhydride or nitrophthalic anhydride of formula II is used as the starting material to react with half molar equivalent of a disubstituted amine NH₂RNH₂ to give a disubstituted phthalic imide which further couples with about equal molar equivalent of an alkali metal sulfide to give a polythioetherimide resin of formula I as shown above. 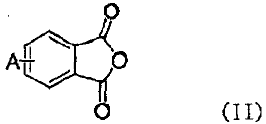 wherein substitute A is chlorine or nitro at 3- or 4-position.

In the third aspect of the invention, a method for preparing a polythioetherimide is provided, wherein chlorophthalic anhydride or nitrophthalic anhydride of the above formula II is used as the starting material to react with half molar equivalent of an organic diamine NH₂RNH₂ to give a disubstituted phthalic imide which further couples with about equal molar equivalent of sulfur to give a polythioetherimide resin of formula I as shown above.

### Brief Description of the Drawings

Fig. 1 shows a reaction process according to one preparation method of the invention.
Fig. 2 shows a reaction process according to another preparation method of the invention.

### Best Embodiments for Carrying out the Invention

After extensive study, the inventors have found a novel polythioctherimide and provided a method for preparing the same. Based on such findings, the invention has been completed.

Various aspects of the invention will be described in detail as follows.

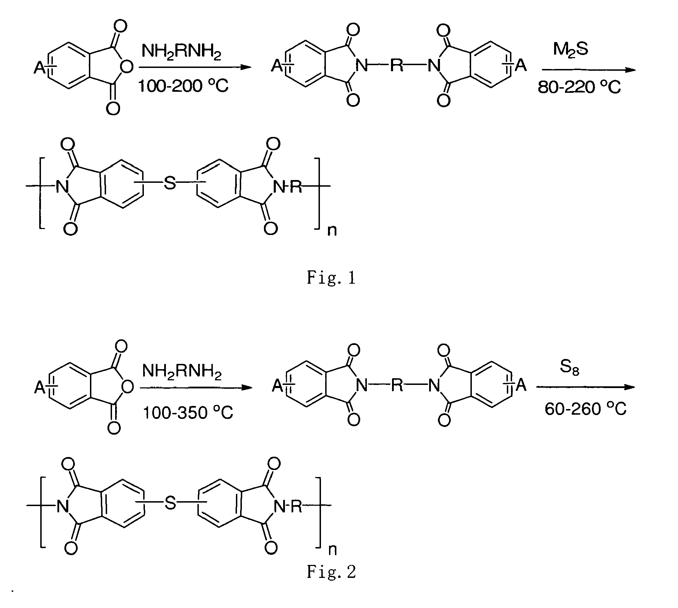

One technical method of preparation according to the invention is illustrated in Fig. 1, wherein chlorophthalic anhydride or nitrophthalic anhydride of formula II is used as the starting material to react with half molar equivalent of a disubstituted amine NH₂RNH₂ to give a disubstituted phthalic imide which further couples with about equal molar equivalent of an alkali metal sulfide to give a polythioetherimide resin of formula I as shown above.

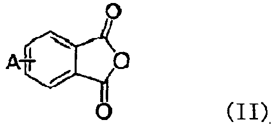

Specifically, in the starting material, i.e. chlorophthalic anhydride or nitrophthalic anhydride, the molar ratio of 3-substituted phthalic anhydride to 4-substituted phthalic anhydride is in any range between 99.9:0.1 and 0.1:99.9.

Specifically, the preparation method is carried out in two steps. The first step involves the reaction between chloro- (or nitro-) phthalic anhydride and half molar equivalent of an organic diamine in a polar non-protonic solvent, or in glacial acetic acid under reflux, or in a mixture of a benzene-type solvent selected from the group consisting of benzene, toluene, xylene and chlorobenzene and a polar non-protonic solvent under reflux, at a temperature ranging from 100°C to 200°C, most preferably from 110°C to 180°C. The second step involves the coupling of the resultant disubstituted phthalic imide with equal molar equivalent of an alkali metal sulfide in a polar non-protonic solvent, or in a mixture of a benzene-type solvent selected from the group consisting of benzene, toluene, xylene and chlorobenzene and a polar non-protonic solvent, optionally with or without the addition of certain catalysts such as sodium hydroxide, potassium hydroxide, anhydrous sodium carbonate, anhydrous potassium carbonate, or anhydrous lithium chloride, at a temperature ranging from 80 °C to 220°C, most preferably from 100°C to 170°C.

Specifically, the polar non-protonic solvent is selected from the group consisting of N,N'-dimethyl formamide (DMF), N,N'-dimethyl acetamide (DMAc), N-methyl-2-pyrrolidone (NMP), dimethyl sulfoxide (DMSO); hexamethylphosphoramide (HMPA) and tetramethylene sulfone.

Specifically, the benzene type solvent refers to benzene, toluene, xylene or chlorobenzene.

Specifically, the alkali metal sulfide is highly pure anhydrous lithium sulfide, potassium sulfide or sodium sulfide, which is usually prepared via two methods, one involving the reaction between an alkali metal and sulfur, and the other involving the purification of an existing industrial grade alkali metal sulfide, particularly sodium sulfide, by heating at high vacuum, or by azeotropic reflux with a benzene type solvent such as benzene, toluene, xylene or chlorobenzene to remove water, or by recrystallization.

Specifically, the organic group R is a substituted or unsubstituted aliphatic or aromatic diamine selected from but not limited to, for example, at least one of the following: 1,6-hexamethylene diamine, 1,6-cyclohexanediamine, p-phenylene diamine, m-phenylene diamine, 4,4'-biphenylene diamine, 3,3'-dimethyl-4,4'-biphenylene diamine, 2,2'-dimethyl-4,4'-biphenylene diamine, 4,4'-diaminodiphenyl ether, 3,4'-diaminodiphenyl ether, 4,4'-diaminobenzophenone, 3,4'-diaminobenzophenone, 4,4'-diaminodiphenyl sulfone, 3,4'-diaminodiphenyl sulfone, 4,4'-diaminodiphenyl methane, 4,4'-diaminodiphenyl isopropane, 4,4'-diaminodiphenyl thioether, 2,2'-dichloro-4,4'-diaminodiphenyl methane, 3,3'-dichloro-4,4'-diaminodiphenyl methane, 4,4'-diaminodiphenoxyl-4",4"'-biphenyl, 4,4'-diaminodiphenoxyl-4",4"'-diphenyl ether, 4,4'-diaminodiphenoxyl-4",4"'-diphenyl sulfone, 4,4'-diaminodiphenoxyl-4",4"'-diphenyl isopropane, 2,4-toluene diamine, 5-methyl-4,6-diethyl-1,3-phenylene diamine, 3,3'-dimethyl-4,4'-diaminodiphenyl methane, or 2,2',3,3'-tetramethyl-4,4'-diaminodiphenyl methane, or mixtures thereof.

Specifically, during the coupling of the disubstituted phthalic imide with the alkali metal sulfide, at least one chain end-capping agent for polymerization can be used to control the polymerization degree and the molecular weight of the final polymer.

Specifically, the chain end-capping agent may be an aromatic compound of formula III,

B-Ar-M III

wherein B may be selected from but not limited to halogen atoms (for example, fluorine, chlorine or bromine, etc.) or nitro, etc.; Ar is a substituted or unsubstituted aromatic group which may be selected from but not limited to one of the following: for example, phenyl, substituted phenyl, biphenyl, substituted biphenyl, furanyl, pyridyl, naphthyl or quinolyl, etc.; and M may be selected from but not limited to one of the following atoms or groups: for example, hydrogen, methyl, acyl, phenyl acyl, alkyl sulphonyl, aromatic sulphonyl, nitro, cyano, azo, carboxyl, trifluoromethyl, imido or substituted imido, etc.. Examples of the chain end-capping agent include 3-chlorophenyl-tert-butyl ketone, 3-fluorophenyl-tert-butyl ketone, 4-chlorobenzophenone, 3-nitrobenzophenone, 4-nitrophenyl methyl sulfone, 4-fluorophenyl phenyl sulfone, 2-iodonitrobenzene, 4-bromophenyl azobenzene, 4-fluoropyridine, 3-chlorobenzoic acid, 1-nitro-4-trifluoromethyl benzene, 1-chloro-3-trifluoromethyl benzene, N -phenyl-3-chlorophthalic imide, N-phenyl-4-fluorophthalic imide, N-methyl-3-chlorophthalic imide, N-methyl-4-nitrophthalic imide, N-butyl-3-chlorophthalic imide, or N-cyclohexyl-4-chlorophthalic imide, etc., or mixtures of two or more thereof , wherein the most preferred molar amount of the chain end-capping agent used is about 0.01-0.15 times that of the corresponding disubstituted phthalic imide.

Another technical method of preparation according to the invention is illustrated in Fig. 2, wherein chlorophthalic anhydride or nitrophthalic anhydride of the above formula II is used as the starting material to react with half molar equivalent of an organic diamine NH₂RNH₂ to give a disubstituted phthalic imide which further couples with about equal molar equivalent of sulfur to give a polythioetherimide resin of formula I as shown above.

Specifically, in the starting material, i.e. chlorophthalic anhydride or nitrophthalic anhydride, the molar ratio of 3-substituted phthalic anhydride to 4-substituted phthalic anhydride is in any range between 99.9:0.1 and 0.1:99.9.

Specifically, the preparation method is carried out in two steps. The first step involves the reaction between a monosubstituted phthalic anhydride and half molar equivalent of an organic diamine in a polar non-protonic solvent, or in glacial acetic acid under reflux, or in a mixture of a benzene-type solvent selected from the group consisting of benzene, toluene, xylene and chlorobenzene and a polar non-protonic solvent under reflux, or in molten state under heating, at a temperature ranging from 100°C to 350°C, most preferably from 120°C to 280°C, to prepare a disubstituted phthalic imide. The second step involves the coupling of the disubstituted phthalic imide with about equal molar equivalent of sulfur in a polar non-protonic solvent or in a mixture of a benzene-type solvent selected from the group consisting of benzene, toluene, xylene and chlorobenzene and a polar non-protonic solvent with the help of a reductant, a catalyst and a reaction aid at a temperature ranging from 60°C to 260 °C, most preferably from 100°C to 190°C, to prepare polythioetherimide, wherein the molar amount of sulfur used is about 0.90-1.30 times, most preferably 0.95-1.15 times that of the corresponding disubstituted phthalic imide.

Specifically, the polar non-protonic solvent is selected from the group consisting of N,N-dimethyl formamide (DMF), N,N-dimethyl acetamide (DMAc), N-methyl-2-pyrrolidone (NMP), dimethyl sulfoxide (DMSO), hexamethylphosphoramide (HMPA), diphenyl sulfone, tetramethylene sulfone and the like.

Specifically, the organic group R is a substituted or unsubstituted aliphatic or aromatic diamine which may be selected from but not limited to, for example, at least one of the following: 1,2-hexanediamine, hexamethylene diamine, 1,6-cyclohexanediamine, p-phenylene diamine, m-phenylene diamine, 4,4'-biphenylene diamine, 3,3'-dimethyl-4,4'-biphenylene diamine, 2,2'-dimethyl-4,4'-biphenylene diamine, 4,4'-diaminodiphenyl ether, 3,4'-diaminodiphenyl ether, 4,4'-diaminobenzophenone, 3,4'-diaminobenzophenone, 4,4'-diaminodiphenyl sulfone, 3,4'-diaminodiphenyl sulfone, 4,4'-diaminodiphenyl methane, 4,4'-diaminodiphenyl isopropane, 4,4'-diaminodiphenyl thioether, 2,2'-dichloro-4,4'-diamino diphenyl methane, 3,3'-dichloro-4,4'-diaminodiphenyl methane, 4,4'-diaminodiphenoxyl-4",4"'-biphenyl, 4,4'-diaminodiphenoxyl-4",4"'-diphenyl ether, 4,4'-diaminodiphenoxyl-4",4"'-diphenyl sulfone, 4,4'-diaminodiphenoxyl-4",4"'-diphenyl isopropane, 2,4-toluene diamine, 5-methyl-4,6-diethyl-1,3-phenylene diamine, 3,3'-dimethyl-4,4'-diaminodiphenyl methane, or 2,2',3,3'-tetramethyl-4,4'-diaminodiphenyl methane and the like, or mixtures thereof.

Specifically, the reductant for the coupling of the disubstituted phthalic imide with sulfur may be selected from but not limited to at least one of the following: formates (for example, sodium formate, potassium formate or lithium formate, etc.), oxalates (for example, sodium oxalate, potassium oxalate or lithium oxalate, etc.), aldehydes (for example, formaldehyde or acetaldehyde, etc.), hydrazines (for example, phenylhydrazine or hydrated hydrazine, etc.), hydroxylamine, elemental metal (for example, iron powder, aluminum powder or zinc powder, etc.), hydrides (for example, sodium hydride, calcium hydride, sodium borohydride or lithium aluminum hydride, etc.), ammonia, hydrogen and the like, or mixtures thereof. The molar amount of the reductant used is 0.2-6 times, most preferably 0.4-3 times that of sulfur.

Specifically, the aid and the catalyst for the coupling polymerization of the disubstituted phthalic imide with sulfur may be selected from but not limited to at least one of the following: carbonates (for example, lithium carbonate, sodium carbonate or potassium carbonate, etc.), hydrocarbonates (for example, sodium hydrocarbonate or potassium hydrocarbonate, etc.), phosphates (for example, sodium hydrophosphate or potassium hydrophosphate, etc.), hydrophosphates (for example, dibasic sodium phosphate or dibasic potassium phosphate, etc.), basic hydroxides (for example, potassium hydroxide, sodium hydroxide or lithium hydroxide, etc.), halides (for example, calcium chloride, sodium chloride, potassium chloride, lithium bromide, potassium fluoride or sodium iodide, etc.) and the like, or mixtures thereof. The molar amount of the aid and the catalyst used is 0.02-3 times, most preferably 0.05-1.5 times that of sulfur.

Specifically, the coupling polymerization of the disubstituted phthalic imide with sulfur may be carried out in inert atmosphere which may be selected from but notlimited to nitrogen, argon and the like.

Specifically, during the coupling polymerization of the disubstituted phthalic imide and sulfur, at least one chain end-capping agent for polymerization can be used to control the polymerization degree and the molecular weight of the final polymer.
Specifically, the chain end-capping agent may be an aromatic compound of formula III,

B-Ar-M III

wherein B may be selected from but not limited to halogen atoms (for example, fluorine, chlorine or bromine, etc.) or nitro, etc.; Ar is a substituted or unsubstituted aromatic group which may be selected from but not limited to one of the following: for example, phenyl, substituted phenyl, biphenyl, substituted biphenyl, furanyl, pyridyl, naphthyl or quinolyl, etc.; and M may be selected from but not limited to one of the following atoms or groups: for example, hydrogen, methyl, acyl, phenyl acyl, alkyl sulphonyl, aromatic sulphonyl, nitro, cyano, azo, carboxyl, trifluoromethyl, imido or substituted imido, etc .. Examples of the chain end-capping agent include 3-chlorophenyl-tert-butyl ketone, 3-fluorophenyl-tert-butyl ketone, 4-chlorobenzophenone, 3-nitrobenzophenone, 4-nitrophenyl methyl sulfone, 4-fluorophenyl phenyl sulfone, 2-iodonitrobenzene, 4-bromophenyl azobenzene, 4-fluoropyridine, 3-chlorobenzoic acid, 1-nitro-4-trifluoromethyl benzene, 1-chloro-3-trifluoromethyl benzene, N -phenyl-3-chlorophthalic imide, N-phenyl-4-fluorophthalic imide, N-methyl-3-chlorophthalic imide, N-methyl-4-nitrophthalic imide, N-butyl-3-chlorophthalic imide, or N-cyclohexyl-4-chlorophthalic imide, etc., or mixtures of two or more thereof , wherein the most preferred molar amount of the chain end-capping agent used is 0.01-0.15 times that of the corresponding disubstituted phthalic imide.

The final polythioetherimides show a logarithmic viscosity number of 0.13dL/g - 1.90dL/g as measured in 0.5g/dL m-cresol at 30°C using Ubbelohde viscometer, and a weight average molecular weight of 3000 - 200000 with respect to polystyrene standard and a polydispersity of 1.8 - 5.4 as measured by gel permeation chromatography.

The final polythioetherimides show a glass transition temperature of about 200 °C-about 350°C according to reheating data as measured by differential scanning calorimetry (DSC) using Perkin Elmer Diamond DSC in nitrogen atmosphere at a heating rate of 20°C/min.

The final polythioetherimides show a viscosity of 500P - 100000P(Poise) as measured at a temperature of 380°C and at a speed of 1000s⁻¹ using Physica MCR-301 rotational rheometer.

The final polythioetherimides show a film tensile strength of 60MPa - 200MPa and a break elongation of 5% - 40% as measured at room temperature and at a speed of 5 mm/min using Instron Model 5567 mechanical tensile tester.

The invention will be described in more detail with reference to the accompanied drawings and the following examples. It should be noted that these examples are intended only to make further illustration of the invention, and should not be construed to limit the claimed scope of the invention in any way.

### Example 1

Into a 2L three-necked flask, dry and clean, were added 182.56g (1.0mol) 4-chlorophthalic anhydride and 1000ml glacial acetic acid, and after they were dissolved under agitation, 113.16g (0.5mol) 3,3'-dimethyl-4,4'-diaminodiphenyl methane (DMMDA) was added. The reactants were heated to 140°C and allowed to react for 24 hours. The reaction solution was cooled to room temperature and then transferred into 10L water. After filtration, a filter cake was obtained as white solid, washed three times with distilled water, and vacuum dried at 120°C to give 249.9g crude dichloromonomer at a yield of 90%. The crude product was recrystallized with dimethyl sulfoxide for use in subsequent polymerization. In argon atmosphere, 27.77g (0.05mol) of the above dichloromonomer, 3.90g (0.05mol) anhydrous sodium sulfide, 2.000g (0.05mol) sodium hydroxide and 300ml N,N'-dimethyl acetamide (DMAc) were added into a 500ml three-necked flask which was dry and clean. The reactants were heated to 120°C and allowed to react for 24 hours. The reaction solution was cooled to room temperature, transferred slowly into 3L water and agitated for 5 hours. After filtration, the resultant filter cake was extracted with 50% ethanol for 12 hours. After vacuum drying at 120°C, 21.1g polyimide was obtained as yellowish powder at a yield of 82%. IR (KBr): 3629, 2922, 1775, 1717, 1604, 1375, 742cm⁻¹. The logarithmic viscosity number was determined to be 0.53dL/g in 0.5g/dL m-cresol at 30°C. The weight average molecular weight and the polydispersity with respect to polystyrene standard were determined to be 38000 and 3.4 respectively by gel permeation chromatography. The glass transition temperature was determined to be 264°C by differential scanning calorimetry (DSC). The film tensile strength and the break elongation were determined to be 92MPa and 17% respectively using a mechanical tensile tester.

### Example 2

Into a 2L three-necked flask, dry and clean, were added 182.56g (1.0mol) of a combination of 3-chlorophthalic anhydride and 4-chlorophthalic anhydride in a mass ratio of 5:1 and 1000ml glacial acetic acid, and after they were dissolved under agitation, 113.16g (0.5mol) DMMDA was added. The reactants were heated to 130 °C and allowed to react for 24 hours. The reaction solution was cooled to room temperature and then transferred into 10L water. After filtration, a filter cake was obtained as white solid, washed three times with distilled water, and vacuum dried at 120°C to give 236g crude dichloromonomer at a yield of 85%. The crude product was recrystallized with a mixed solvent of DMAc and toluene (volume ratio 2:1) for use in subsequent polymerization. In nitrogen atmosphere, 27.77g (0.05mol) of the above dichloromonomer, 3.90g (0.05mol) anhydrous sodium sulfide, 6.36g (0.06mol) anhydrous sodium carbonate and 250ml DMAc were added into a 500ml three-necked flask which was dry and clean. The reactants were heated to 130°C and allowed to react for 36 hours. The reaction solution was cooled to room temperature, transferred slowly into 2L water and agitated for 10 hours. After filtration, the resultant filter cake was extracted with 50% ethanol for 14 hours. After vacuum drying at 120°C, 22.1g polyimide was obtained as yellowish powder at a yield of 86%. IR (KBr): 3476, 1775, 1716, 1606, 1374, 744cm⁻¹. The logarithmic viscosity number was determined to be 0.24dL/g in 0.5g/dL m-cresol at 30°C. The weight average molecular and the polydispersity weight with respect to polystyrene standard were determined to be 16000 and 2.8 respectively by gel permeation chromatography. The glass transition temperature was determined to be 272°C by differential scanning calorimetry (DSC). The film tensile strength and the break elongation were determined to be 104MPa and 13% respectively using a mechanical tensile tester.

### Example 3

Into a 1L three-necked flask, dry and clean, were added 91.28g (0.5mol) of a combination of 3-chlorophthalic anhydride and 4-chlorophthalic anhydride in a mass ratio of 3:1 and 500ml DMAc, and after they were dissolved under agitation, 49.56g (0.25mol) 4,4'-diaminodiphenyl methane (MDA) was added. The reactants were first heated to 80°C and allowed to react for 2 hours, and then heated to 130°C and allowed to react for 16 hours. The reaction solution was concentrated by depressurization to 200ml and then transferred into 3L water. After filtration, a filter cake was obtained as white solid, washed three times with distilled water, and vacuum dried at 120°C to give 116g crude dichloromonomer at a yield of 88%. The crude product was vacuum melted for use in subsequent polymerization. In nitrogen atmosphere, 26.37g (0.05mol) of the above dichloromonomer, 2.30g (0.05mol) anhydrous lithium sulfide and 200ml N-methyl-2-pyrrolidone (NMP) were added into a 500ml three-necked flask which was dry and clean. The reactants were heated to 170°C and allowed to react for 8 hours. The reaction solution was cooled to room temperature, transferred slowly into 2L water and agitated for 12 hours. After filtration, the resultant filter cake was extracted with 90% ethanol for 24 hours. After vacuum drying at 150°C, 21.0g polyimide was obtained as yellowish powder at a yield of 86%. IR (KBr): 3438, 1778, 1716, 1606, 1378, 741cm⁻¹. The logarithmic viscosity number was determined to be 0.37dL/g in 0.5g/dL m-cresol at 30°C . The weight average molecular weight and the polydispersity with respect to polystyrene standard were determined to be 28000 and 4.1 respectively by gel permeation chromatography. The glass transition temperature was determined to be 275 °C by differential scanning calorimetry (DSC). The viscosity was determined to be 7600P(Poise) at a temperature of 380°C and at a speed of 1000S⁻¹ using Physica MCR-301 rotational rheometer. The film tensile strength and the break elongation were determined to be 85MPa and 6% respectively using a mechanical tensile tester.

### Example 4

Into a 1L three-necked flask, dry and clean, were added 91.28g (0.5mol) of a combination of 3-chlorophthalic anhydride and 4-chlorophthalic anhydride in a mass ratio of 2:1, 400ml DMF and 50ml toluene, and after they were dissolved under agitation, 50.06g (0.25mol) 4,4'-diaminodiphenyl ether (ODA) was added. The reactants were first heated to 90°C and allowed to react for 2 hours, and then heated to 150°C and allowed to react for 18 hours. The reaction solution was concentrated by depressurization to 150ml and then transferred into 2L water. After filtration, a filter cake was obtained as white solid, washed three times with distilled water, and vacuum dried at 100°C to give 119g crude dichloromonomer at a yield of 90%. The crude product was recrystallized with DMSO for use in subsequent polymerization. In argon atmosphere, 26.46g (0.05mol) of the above dichloromonomer, 2.30g (0.05mol) anhydrous lithium sulfide, 6.91g (0.05mol) anhydrous potassium carbonate and 200ml DMSO were added into a 500ml three-necked flask which was dry and clean. The reactants were heated to 100°C and allowed to react for 18 hours. The reaction solution was cooled to room temperature, transferred slowly into 1L water and agitated for 10 hours. After filtration, the resultant filter cake was extracted with 90% methanol for 24 hours. After vacuum drying at 150°C, 20.3g polyimide was obtained as yellowish powder at a yield of 83%. IR (KBr): 3488, 1774, 1716, 1603, 1377, 742cm⁻¹. The logarithmic viscosity number was determined to be 0.48dL/g in 0.5g/dL m-cresol at 30°C. The weight average molecular weight and the polydispersity with respect to polystyrene standard were determined to be 31000 and 3.9 respectively by gel permeation chromatography. The glass transition temperature was determined to be 268°C by differential scanning calorimetry (DSC). The viscosity was determined to be 9000P at a temperature of 380°C and at a speed of 1000S⁻¹ using Physica MCR-301 rotational rheometer. The film tensile strength and the break elongation were determined to be 106MPa and 18% respectively using a mechanical tensile tester.

### Example 5

Into a 3L three-necked flask, dry and clean, were added 273.39g (1.5mol) of a combination of 3-chlorophthalic anhydride and 4-chlorophthalic anhydride in a mass ratio of 1:1, 1000ml DMAc and 1000ml xylene, and after they were dissolved under agitation, 81.10g (0.75mol) p-phenylene diamine was added. The reactants were first allowed to react at 80°C for 2 hours, and then heated to 160°C and allowed to react for 24 hours under reflux with water removed. The reaction solution was concentrated by depressurization to about 800ml and then transferred into 12L water. After filtration, a filter cake was obtained, washed three times with distilled water, and vacuum dried at 100°C to give 292g crude dichloromonomer at a yield of 89%. The crude product was recrystallized with a mixed solvent of DMSO and toluene for use in subsequent polymerization. In argon atmosphere, 43.72g (0.1mol) of the above dichloromonomer, 7.80g (0.1mol) anhydrous sodium sulfide and 450ml N-methyl-2-pyrrolidone (NMP) were added into a 1L three-necked flask which was dry and clean. The reactants were heated to 160°C and allowed to react for 30 hours. The reaction solution was cooled to room temperature, transferred slowly into 4L water and agitated for 12 hours. After filtration, the resultant filter cake was extracted with 90% methanol for 24 hours. After vacuum drying at 120°C, 35.8g polyimide was obtained as yellowish powder at a yield of 90%. IR (KBr): 3442, 1779, 1714, 1601, 1382, 739cm⁻¹. The logarithmic viscosity number was determined to be 0.68dL/g in 0.5g/dL m-cresol at 30°C. The weight average molecular weight and the polydispersity with respect to polystyrene standard were determined to be 35000 and 3.5 respectively by gel permeation chromatography. The glass transition temperature was determined to be 296°C by differential scanning calorimetry (DSC). The film tensile strength and the break elongation were determined to be 159MPa and 12% respectively using a mechanical tensile tester.

### Example 6

Into a 1L three-necked flask, dry and clean, were added 32.86g (0.18mol) 4-chlorophthalic anhydride, 3.65g (0.02mol) 3-chlorophthalic anhydride and 400ml glacial acetic acid, and after they were dissolved under agitation, 19.83g (0.1mol) 4,4'-diaminodiphenyl methane was added. The reactants were heated to 140°C and allowed to react for 24 hours under reflux. The reaction solution was cooled to room temperature. After filtration, a filter cake was obtained, washed three times with distilled water, and vacuum dried at 120°C to give 48.52g crude dichlorophthalic imide at a yield of 92%. The crude product was recrystallized with a mixed solvent of toluene and N,N-dimethylacetamide (4:1, v/v) for use in subsequent polymerization. In argon atmosphere, 7.9107g (0.015mol) of the above dichloromonomer, 0.4800g (0.015mol) sulfur, 1.3241g (0.035mol) sodium borohydride, 1.7954g (0.032mol) potassium hydroxide, 0.4439g (0.004mol) calcium chloride and 150ml N,N-dimethylacetamide were added into a 500ml three-necked flask which was dry and clean. The reactants were heated to 150°C under agitation and allowed to react for 8 hours. The reaction solution was cooled to room temperature, transferred slowly into 2L water and agitated for 12 hours. After filtration, the resultant filter cake was washed three times with distilled water, and then extracted with 95% ethanol for 24 hours. After vacuum drying at 120°C, 6.89g polyimide was obtained as white powder at a yield of 94%. IR (KBr): 2935, 1785, 1720, 1609, 1385, 728cm⁻¹. The logarithmic viscosity number was determined to be 1.26dL/g in 0.5g/dL m-cresol at 30°C. The weight average molecular weight and the polydispersity with respect to polystyrene standard were determined to be 88000 and 3.4 respectively by gel permeation chromatography. The glass transition temperature was determined to be 275°C by differential scanning calorimetry (DSC). The viscosity was determined to be 60000P at a temperature of 380°C and at a speed of 1000S⁻¹ using Physica MCR-301 rotational rheometer. The film tensile strength and the break elongation were determined to be 126MPa and 9% respectively using a mechanical tensile tester.

### Example 7

Into a 500ml three-necked flask, dry and clean, were added 91.28g (0.50mol) 4-chlorophthalic anhydride, 91.28g (0.50mol) 3-chlorophthalic anhydride and 100.12g (0.5mol) 4,4'-diaminodiphenyl ether. They were heated slowly under vacuum to 260°C until a homogenous melt was obtained, and then allowed to react for 4 hours under agitation. After cooled to room temperature, 259.38g crude dichlorophthalic imide was obtained at a yield of 98%. The crude product was used in subsequent polymerization as it was. Into a 500ml three-necked flask, dry and clean, were added 10.5872g (0.020mol) of the above dichloromonomer, 0.6602g (0.0206mol) sulfur, 0.9600g (0.04mol) sodium hydride, 2.8471g (0.0206mol) potassium carbonate, 0.2120g (0.005mol) lithium chloride and 180ml N-methylpyrrolidone. The reactants were heated to 80°C under agitation and allowed to react for 24 hours, and then 0.3092g (0.0012mol) N-phenyl-3-chlorophthalic imide was added and allowed to react for 4 hours. The reaction solution was cooled to room temperature, transferred slowly into 2L water and agitated for 12 hours. After filtration, the resultant filter cake was washed three times with distilled water, and then extracted with 95% ethanol for 24 hours. After vacuum drying at 120°C, 9.26g polyimide was obtained as yellowish powder at a yield of 92%. IR (KBr): 2925, 1783, 1718, 1601, 1384, 725cm⁻¹. The logarithmic viscosity number was determined to be 0.88dL/g in 0.5g/dL m-cresol at 30°C. The weight average molecular weight and the polydispersity with respect to polystyrene standard were determined to be 62000 and 3.8 respectively by gel permeation chromatography. The glass transition temperature was determined to be 272 °C by differential scanning calorimetry (DSC). The viscosity was determined to be 8000P at a temperature of 380°C and at a speed of 1000S⁻¹ using Physica MCR-301 rotational rheometer. The film tensile strength and the break elongation were determined to be 119MPa and 16% respectively using a mechanical tensile tester.

### Example 8

Into a 3L three-necked flask, dry and clean, were added 28.97g (0.15mol) 4-nitrophthalic anhydride, 9.65g (0.05mol) 3-nitrophthalic anhydride and 1500ml xylene, and after they were dissolved under agitation, 19.83g (0.10mol) 4,4'-diaminodiphenyl methane was added. The reactants were heated to 160°C and allowed to react for 15 hours under reflux. The reaction solution was cooled to room temperature. After filtration, a filter cake was obtained, washed three times with distilled water, and vacuum dried at 120°C to give 48.82g crude dinitrophthalic imide at a yield of 89%. The crude product was recrystallized with a mixed solvent of toluene and N,N-dimethylformamide (4:1, v/v) for use in subsequent polymerization. In nitrogen atmosphere, 16.4520g (0.030mol) of the above dinitromonomer, 0.9618g (0.030mol) sulfur, 0.9909g (0.030mol) hydroxylamine, 2.0732g (0.015mol) potassium carbonate, 0.0424g (0.001mol) lithium chloride and 300ml dimethylsulfoxide were added into a 1L three-necked flask which was dry and clean. The reactants were heated to 110°C under agitation and allowed to react for 8 hours. The reaction solution was concentrated to about 100ml, transferred slowly into 1000ml distilled water and agitated for 12 hours. After filtration, the resultant filter cake was washed three times with distilled water, and then extracted with 95% ethanol for 24 hours. After vacuum drying at 120°C, 13.92g polyimide was obtained as white powder at a yield of 94%. IR (KBr): 2960, 1788, 1721, 1605, 1383, 721cm⁻¹. The logarithmic viscosity number was determined to be 0.59dL/g in 0.5g/dL m-cresol at 30°C. The weight average molecular weight and the polydispersity with respect to polystyrene standard were determined to be 36000 and 2.8 respectively by gel permeation chromatography. The glass transition temperature was determined to be 278°C by differential scanning calorimetry (DSC). The viscosity was determined to be 6000P at a temperature of 380°C and at a speed of 10005⁻¹ using Physica MCR-301 rotational rheometer. The film tensile strength and the break elongation were determined to be 106MPa and 10% respectively using a mechanical tensile tester.

### Example 9

Into a 2L three-necked flask, dry and clean, were added 38.62g (0.20mol) 4-nitrophthalic anhydride, 38.62g (0.20mol) 3-nitrophthalic anhydride and 800ml N,N-dimethylacetamide, and after they were dissolved under agitation, 42.45g (0.20mol) 4,4'-diaminobenzophenone was added. The reactants were heated to 170 °C and allowed to react for 15 hours under reflux. The reaction solution was cooled to room temperature. After filtration, a filter cake was obtained, washed three times with anhydrous ethanol, and vacuum dried at 120°C to give 106.87g crude dinitrophthalic imide at a yield of 95%. The crude product was recrystallized with a mixed solvent of toluene and N,N-dimethylformamide (2:1, v/v) for use in subsequent polymerization. In argon atmosphere, 8.4371g (0.015mol) of the above dinitromonomer, 0.4905g (0.0153mol) sulfur, 1.3241g (0.035mol) sodium borohydride, 1.7955g (0.032mol) potassium hydroxide , 0.7495g (0.005mol) sodium iodide, 120ml N,N-dimethylacetamide and 15ml xylene were added into a 500ml three-necked flask which was dry and clean. The reactants were heated to 150°C under agitation and allowed to react for 16 hours. The reaction solution was concentrated to about 100ml, transferred slowly into 1000ml distilled water and agitated for 12 hours. After filtration, the resultant filter cake was washed three times with distilled water, and then extracted with 95% ethanol for 24 hours. After vacuum drying at 120°C, 7.24g polyimide was obtained as yellowish powder at a yield of 96%. IR (KBr): 3060, 1670, 1784, 1718, 1600, 1388, 718cm⁻¹. The logarithmic viscosity number was determined to be 0.68dL/g in 0.5g/dL m-cresol at 30°C . The weight average molecular weight and the polydispersity with respect to polystyrene standard were determined to be 42000 and 3.1 respectively by gel permeation chromatography. The glass transition temperature was determined to be 288°C by differential scanning calorimetry (DSC). The viscosity was determined to be 6800P at a temperature of 380°C and at a speed of 1000S⁻¹ using Physica MCR-301 rotational rheometer. The film tensile strength and the break elongation were determined to be 136MPa and 17% respectively using a mechanical tensile tester.

### Example 10

Into a 3L three-necked flask, dry and clean, were added 115.87g (0.60mol) 4-nitrophthalic anhydride, 77.24g (0.40mol) 3-nitrophthalic anhydride and 1800ml glacial acetic acid, and after they were dissolved under agitation, 113.16g (0.50mol) 3,3'-dimethyl-4,4'-diaminodiphenyl methane was added. The reactants were heated to 130°C and allowed to react for 24 hours under reflux. The reaction solution was cooled to room temperature, transferred into 8L distilled water and agitated for 3 hours. After filtration, a filter cake was obtained, washed three times with distilled water, and vacuum dried at 120°C to give 265.21g crude dinitrophthalic imide at a yield of 92%. The crude product was vacuum melted for use in subsequent polymerization. Into a 100ml three-necked flask, dry and clean, were added 2.8827g (0.005mol) of the above dinitromonomer, 0.1664g (0.0052mol) sulfur, 0.5518g (0.0052mol) benzaldehyde, 0.5511g (0.0052mol) sodium carbonate, 1.3318g (0.012mol) calcium chloride, 0.1053g (0.0004mol) 3-nitrodiphenyl sulfone and 50ml N-methylpyrrolidone. The reactants were heated to 140°C under agitation and allowed to react for 10 hours. The reaction solution was cooled to room temperature, transferred slowly into 500ml distilled water and agitated for 12 hours. After filtration, the resultant filter cake was washed three times with distilled water, and then extracted with 95% ethanol for 24 hours. After vacuum drying at 120°C, 2.39g polyimide was obtained as white powder at a yield of 89%. IR (KBr): 2928, 1782, 1724, 1603, 1381, 725cm⁻¹. The logarithmic viscosity number was determined to be 1.20dL/g in 0.5g/dL m-cresol at 30°C. The weight average molecular weight and the polydispersity with respect to polystyrene standard were determined to be 72000 and 3.9 respectively by gel permeation chromatography. The glass transition temperature was determined to be 264°C by differential scanning calorimetry (DSC). The film tensile strength and the break elongation were determined to be 128MPa and 13% respectively using a mechanical tensile tester.

### Example 11

Into a 2L three-necked flask, dry and clean, were added 18.26g (0.10mol) 4-chlorophthalic anhydride, 91.28g (0.50mol) 3-chlorophthalic anhydride and 1000ml glacial acetic acid, and after they were dissolved under agitation, 76.31g (0.30mol) 2,2',3,3'-tetramethyl-4,4'-diaminodiphenyl methane was added. The reactants were heated to 130°C and allowed to react for 28 hours under reflux. The reaction solution was cooled to room temperature, transferred into 5L distilled water and agitated for 3 hours. After filtration, a filter cake was obtained, washed three times with distilled water, and vacuum dried at 120°C to give 161.04g crude dichlorophthalic imide at a yield of 92%. The crude product was recrystallized with a mixed solvent of toluene and N,N-dimethylformamide (2:1, v/v) for use in subsequent polymerization. In nitrogen atmosphere, 2.9175g (0.005mol) of the above dichloromonomer, 0.1600g (0.005mol) sulfur, 0.6700g (0.005mol) sodium oxalate, 1.0600g (0.010mol) sodium carbonate, 0.0424g (0.001 mol) lithium chloride and 60ml tetramethylene sulfone were added into a 100ml three-necked flask which was dry and clean. The reactants were heated to 180°C under agitation and allowed to react for 24 hours. The reaction solution was cooled to room temperature, transferred slowly into 500ml methanol and agitated for 12 hours. After filtration, the resultant filter cake was washed three times with distilled water, and then extracted with 95% ethanol for 24 hours. After vacuum drying at 120°C, 2.43g polyimide was obtained as white powder at a yield of 93%. IR (KBr): 2940, 1786, 1721, 1608, 1380, 726cm⁻¹. The logarithmic viscosity number was determined to be 1.73dL/g in 0.5g/dL m-cresol at 30°C. The weight average molecular weight and the polydispersity with respect to polystyrene standard were determined to be 135000 and 4.4 respectively by gel permeation chromatography. The glass transition temperature was determined to be 292°C by differential scanning calorimetry (DSC). The film tensile strength and the break elongation were determined to be 133MPa and 9% respectively using a mechanical tensile tester.

## Claims

1. A polythioetherimide, wherein the polythioetherimide has a structure of formula I: 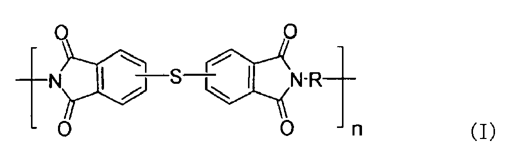 wherein thioether bond is located at 3-position or 4-position;
wherein the organic group R is a substituted or unsubstituted aliphatic or aromatic diamine; and
wherein said polythioetherimide has a logarithmic viscosity number of 0.13 dL/g to 1.90 dL/g measured in 0.5 g/dL m-cresol at 30°C, a weight average molecular weight of 3.000 to 200, 000 and a polydispersity of 1.8 to 5.4.

2. The polythioetherimide of claim 1, wherein the organic group R is selected from the group consisting of 1,6-hexamethylene diamine, 1,6-cyclohexanediamine, p-phenylene diamine, m-phenylene diamine, 4,4'-biphenylene diamine, 3,3'-dimethyl-4,4'-biphenylene diamine, 2,2'-dimethyl-4,4'-biphenylene diamine, 4,4'-diaminodiphenyl ether, 3,4'-diaminodiphenyl ether, 4,4'-diaminobenzophenone, 3,4'-diaminobenzophenone, 4,4'-diaminodiphenyl sulfone, 3,4'-diaminodiphenyl sulfone, 4,4'-diaminodiphenyl methane, 4,4'-diaminodiphenyl isopropane, 4,4'-diaminodiphenyl thioether, 2,2'-dichloro-4,4'-diaminodiphenyl methane, 3,3'-dichloro-4,4'-diaminodiphenyl methane, 4,4'-diaminodiphenoxyl-4",4"'-biphenyl, 4,4'-diaminodiphenoxyl-4",4"'-diphenyl ether, 4,4'-diaminodiphenoxyl-4",4"'-diphenyl sulfone, 4,4'-diaminodiphenoxyl-4",4"'-diphenyl isopropane, 2,4-toluene diamine, 5-methyl-4,6-diethyl-1,3-phenylene diamine, 3,3'-dimethyl-4,4'-diaminodiphenyl methane, or 2,2',3,3'-tetramethyl-4,4'-diaminodiphenyl methane, or mixtures thereof.

3. A method for preparing a polythioetherimide, wherein chlorophthalic anhydride or nitrophthalic anhydride isomer of formula II is used as the starting material to react with half molar equivalent of a disubstituted amine NH₂RNH₂ to give a disubstituted phthalic imide which further couples with about equal molar equivalent of an alkali metal sulfide to give a polythioetherimide resin of formula I as shown above, 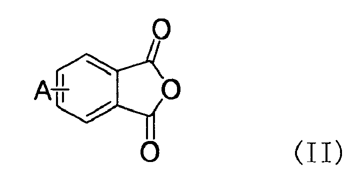 wherein substitute A is chlorine or nitro at 3- or 4-position.

4. The preparation method of Claim 3, wherein the molar ratio of 3-substituted phthalic anhydride to 4-substituted phthalic anhydride in the starting material, i.e. chlorophthalic anhydride or nitrophthalic anhydride, is in the range between 99.9:0.1 and 0.1:99.9, or wherein the preparation method is carried out in two steps, wherein the first step involves the reaction between the chlorophthalic anhydride or nitrophthalic anhydride isomer and half molar equivalent of a disubstituted amine NH₂RNH₂ in a polar non-protonic solvent, or in glacial acetic acid under reflux, or in a mixture of a benzene-type solvent selected from the group consisting of benzene, toluene, xylene and chlorobenzene_and a polar non-protonic solvent under reflux, at a temperature ranging from 100°C to 200°C, most preferably from 110°C to 180°C; and the second step involves the coupling reaction of the resultant disubstituted phthalic imide with equal molar equivalent of an alkali metal sulfide in a polar non-protonic solvent, or in a mixture of a benzene type solvent selected from the group consisting of benzene toluene, xylene and chlorobenzene and a polar non-protonic solvent, in the presence or absence of an optional catalyst such as sodium hydroxide, potassium hydroxide, anhydrous sodium carbonate, anhydrous potassium carbonate or anhydrous lithium chloride, at a temperature ranging from 80°C to 220°C, most preferably from 100°C to 170°C.

5. The preparation method of Claim 4, wherein the polar non-protonic solvent is N,N'-dimethyl formamide (DMF), N,N'-dimethyl acetamide (DMAc), N-methyl-2-pyrrolidone (NMP), dimethyl sulfoxide (DMSO), hexamethylphosphoramide (HMPA) or tetramethylene sulfone, or wherein the benzene type solvent is selected from the group consisting of benzene, toluene, xylene and chlorobenzene, wherein the alkali metal sulfide is anhydrous lithium sulfide, potassium sulfide or sodium sulfide; and preferably, the alkali metal sulfide is prepared via one of the following two methods:
(i) reacting an alkali metal with sulfur;
(ii) purifying an industrial grade alkali metal sulfide, particularly sodium sulfide, by heating at high vacuum, or by azeotropic reflux with a benzene type solvent selected from the group consisting of benzene, toluene, xylene and chlorobenzene to remove water, or by recrystallization or
wherein the organic group R is a substituted or unsubstituted aliphatic or aromatic diamine.

6. The preparation method according to claim 5 wherein the organic group R is selected from the group consisting of 1,6-hexamethylene diamine, 1,6-cyclohexanediamine, p-phenylene diamine, m-phenylene diamine, 4,4'-biphenylene diamine, 3,3'-dimethyl-4,4'-biphenylene diamine, 2,2'-dimethyl-4,4'-biphenylene diamine, 4,4'-diaminodiphenyl ether, 3,4'-diaminodiphenyl ether, 4,4'-diaminobenzophenone, 3,4'-diaminobenzophenone, 4,4'-diaminodiphenyl sulfone, 3,4'-diaminodiphenyl sulfone, 4,4'-diaminodiphenyl methane, 4,4'-diaminodiphenyl isopropane, 4,4'-diaminodiphenyl thioether, 2,2'-dichloro-4,4'-diaminodiphenyl methane, 3,3'-dichloro-4,4'-diaminodiphenyl methane, 4,4'-diaminodiphenoxyl-4",4"'-biphenyl, 4,4'-diaminodiphenoxyl-4",4"'-diphenyl ether, 4,4'-diaminodiphenoxyl-4",4"'-diphenyl sulfone, 4,4'-diaminodiphenoxyl-4",4"'-diphenyl isopropane, 2,4-toluene diamine, 5-methyl-4,6-diethyl-1,3-phenylene diamine, 3,3'-dimethyl-4,4'-diaminodiphenyl methane, or 2,2',3,3'-tetramethyl-4,4'-diaminodiphenyl methane, or mixtures thereof.

7. The preparation method of Claim 4, wherein,
using at least one chain end-capping agent for polymerization to control the polymerization degree and the molecular weight of the final polymer during the coupling reaction of the disubstituted phthalic imide with the alkali metal sulfide.

8. The preparation method of Claim 7, wherein the chain end-capping agent is an aromatic compound of formula III,
B-Ar-M III
wherein B is selected from but not limited to halogen atoms such as fluorine, chlorine or bromine, or nitro group; Ar is a substituted or unsubstituted aromatic group which may be selected from but not limited to one of the following: phenyl, substituted phenyl, biphenyl, substituted biphenyl, furanyl, pyridyl, naphthyl or quinolyl, etc.; M may be selected from but not limited to one of the following atoms or groups: for example, hydrogen, methyl, acyl, phenyl acyl, alkyl sulphonyl, aromatic sulphonyl, nitro, cyano, azo, carboxyl, trifluoromethyl, imido or substituted imido; and preferably, the chain end-capping agent is 3-chlorophenyl-tert-butyl ketone, 3-fluorophenyl-tert-butyl ketone, 4-chlorobenzophenone, 3-nitrobenzophenone, 4-nitrophenyl methyl sulfone, 4-fluorophenyl phenyl sulfone, 2-iodonitrobenzene, 4-bromophenyl azobenzene, 4-fluoropyridine, 3-chlorobenzoic acid, 1-nitro-4-trifluoromethyl benzene, 1-chloro-3-trifluoromethyl benzene, N-phenyl-3-chlorophthalic imide, N-phenyl-4-fluorophthalic imide, N-methyl-3-chlorophthalic imide, N-methyl-4-nitrophthalic imide, N-butyl-3-chlorophthalic imide, or N-cyclohexyl-4-chlorophthalic imide, or mixtures of two or more thereof , wherein the most preferred molar amount of the chain end-capping agent used is 0.01-0.15 times that of the corresponding disubstituted phthalic imide.

9. A method for preparing a polythioetherimide, wherein chlorophthalic anhydride or nitrophthalic anhydride of the above formula II is used as the starting material to react with half molar equivalent of an organic diamine NH₂RNH₂ to give a disubstituted phthalic imide which further couples with about equal molar equivalent of sulfur to give a polythioetherimide resin of formula I as shown above.

10. The preparation method of Claim 9, wherein the molar ratio of 3-substituted phthalic anhydride to 4-substituted phthalic anhydride in the starting material, i.e. chlorophthalic anhydride or nitrophthalic anhydride, is in any range between 99.9:0.1 and 0.1:99.9, or
wherein the preparation method is carried out in two steps, wherein the first step involves the reaction between isomeric chlorophthalic anhydride or nitrophthalic anhydride and half molar equivalent of an organic diamine in a polar non-protonic solvent, or in glacial acetic acid under reflux, or in a mixture of a benzene type solvent selected from the group consisting of benzene, toluene, xylene and chlorobenzene_and a polar non-protonic solvent under reflux, or in molten state under heating, at a temperature ranging from 100°C to 350 °C, most preferably from 120°C to 280°C, to produce a disubstituted phthalic imide; and the second step involves the coupling reaction of the disubstituted phthalic imide with about equal molar equivalent of sulfur in a polar non-protonic solvent or in a mixture of a benzene-type solvent selected from the group consisting of benzene, toluene, xylene and chlorobenzene and a polar non-protonic solvent in the presence of a reductant, a catalyst and a reaction aid at a temperature ranging from 60°C to 260 °C, most preferably from 100°C to 190°C, to produce polythioetherimide, wherein the molar amount of sulfur used is 0.90-1.30 times, most preferably 0.95-1.15 times that of the corresponding disubstituted phthalic imide.

11. The preparation method of Claim 10, wherein the polar non-protonic solvent is N,N-dimethyl formamide (DMF), N,N-dimethyl acetamide (DMAc), N-methyl-2-pyrrolidone (NMP), dimethyl sulfoxide (DMSO), hexamethylphosphoramide (HMPA), diphenyl sulfone or tetramethylene sulfone.

12. The preparation method of Claim 10, wherein the benzene type solvent is selected from the group consisting of_benzene, toluene, xylene and chlorobenzene,
wherein the organic group R is a substituted or unsubstituted aliphatic or
aromatic diamine which may be selected from but not limited to, for example, at least one of the following: 1,2-hexanediamine, 1,6-hexamethylene diamine, 1,6-cyclohexanediamine, p-phenylene diamine, m-phenylene diamine, 4,4'-biphenylene diamine, 3,3'-dimethyl-4,4'-biphenylene diamine, 2,2'-dimethyl-4,4'-biphenylene diamine, 4,4'-diaminodiphenyl ether, 3,4'-diaminodiphenyl ether, 4,4'-diaminobenzophenone, 3,4'-diaminobenzophenone, 4,4'-diaminodiphenyl sulfone, 3,4'-diaminodiphenyl sulfone, 4,4'-diaminodiphenyl methane, 4,4'-diaminodiphenyl isopropane, 4,4'-diaminodiphenyl thioether, 2,2'-dichloro-4,4'-diamino diphenyl methane, 3,3'-dichloro-4,4'-diaminodiphenyl methane, 4,4'-diaminodiphenoxyl-4",4"'-biphenyl, 4,4'-diaminodiphenoxyl-4",4"'-diphenyl ether, 4,4'-diaminodiphenoxyl-4",4"'-diphenyl sulfone, 4,4'-diaminodiphenoxyl-4",4"'-diphenyl isopropane, 2,4-toluene diamine, 5-methyl-4,6-diethyl-1,3-phenylene diamine, 3,3'-dimethyl-4,4'-diaminodiphenyl methane, or 2,2',3,3'-tetramethyl-4,4'-diaminodiphenyl methane, or mixtures thereof, or
wherein the reductant for the coupling of the disubstituted phthalic imide with sulfur may be selected from but not limited to at least one of the following: formates (such as sodium formate, potassium formate or lithium formate.), oxalates (such as sodium oxalate, potassium oxalate or lithium oxalate), aldehydes (such as formaldehyde or acetaldehyde), hydrazines (such as phenylhydrazine or hydrated hydrazine), hydroxylamine, elemental metal (such as iron powder, aluminum powder or zinc powder), hydrides (such as sodium hydride, calcium hydride, sodium borohydride or lithium aluminum hydride), ammonia, hydrogen and the like, or mixtures thereof; and the molar amount of the reductant used is 0.2-6 times, most preferably 0.4-3 times that of sulfur, or wherein the aid and the catalyst for the coupling reaction of the disubstituted phthalic imide with sulfur may be selected from but not limited to at least one of the following: carbonates such as lithium carbonate, sodium carbonate or potassium carbonate, hydrocarbonates such as sodium hydrocarbonate or potassium hydrocarbonate, phosphates such as sodium hydrophosphate or potassium hydrophosphate, hydrophosphates such as dibasic sodium phosphate or dibasic potassium phosphate, basic hydroxides such as potassium hydroxide, sodium hydroxide or lithium hydroxide, halides such as calcium chloride, sodium chloride, potassium chloride, lithium bromide, potassium fluoride or sodium iodide, or mixtures thereof; and
the molar amount of the aid and the catalyst used is 0.02-3 times, most preferably 0.05-1.5 times that of sulfur, or
wherein the coupling polymerization of the disubstituted phthalic imide with sulfur is carried out in inert atmosphere which may be selected from but not limited to nitrogen or argon.

13. The preparation method of Claim 10, wherein, using at least one chain end-capping agent for polymerization to control the polymerization degree and the molecular weight of the final polymer during the coupling reaction of the disubstituted phthalic imide and sulfur.

14. The preparation method of Claim 13, wherein the chain end-capping agent is an aromatic compound of formula III,
B-Ar-M III
wherein B may be selected from but not limited to halogen atoms such as fluorine, chlorine or bromine, or nitro, etc.; Ar is a substituted or unsubstituted aromatic group which may be selected from but not limited to one of the following: phenyl, substituted phenyl, biphenyl, substituted biphenyl, furanyl, pyridyl, naphthyl or quinolyl; M may be selected from but not limited to one of the following atoms or groups: for example, hydrogen, methyl, acyl, phenyl acyl, alkyl sulphonyl, aromatic sulphonyl, nitro, cyano, azo, carboxyl, trifluoromethyl, imido or substituted imido; and
preferably, the chain end-capping agent is 3-chlorophenyl-tert-butyl ketone, 3-fluorophenyl-tert-butyl ketone, 4-chlorobenzophenone, 3-nitrobenzophenone, 4-nitrophenyl methyl sulfone, 4-fluorophenyl phenyl sulfone, 2-iodonitrobenzene, 4-bromophenyl azobenzene, 4-fluoropyridine, 3-chlorobenzoic acid, 1-nitro-4-trifluoromethyl benzene, 1-chloro-3-trifluoromethylbenzene, N-phenyl-3-chlorophthalic imide, N-phenyl-4-fluorophthalic imide, N-methyl-3-chlorophthalic imide, N-methyl-4-nitrophthalic imide, N-butyl-3-chlorophthalic imide, or N-cyclohexyl-4-chlorophthalic imide, etc., or mixtures of two or more thereof , wherein the most preferred molar amount of the chain end-capping agent used is 0.01-0.15 times that of the corresponding disubstituted phthalic imide.

15. The preparation method of any one of Claims 3 to 14, wherein the polythioetherimide has a logarithmic viscosity number of 0.13dL/g to 1.90dL/g as measured in 0.5 g/dL m-cresol at 30°, or wherein the polythioetherimide has a weight average molecular weight of 3,000 to 200,000 and a polydispersity of 1.8 to 5.4, or wherein the polythioetherimide has a glass transition temperature of 200°C to 350°C according to reheating data, or
wherein the polythioetherimide has a viscosity of 500P to 100,000P as measured at a temperature of 380°C and at a speed of 1000 s⁻¹, or
wherein the polythioetherimide has a film tensile strength of 60MPa to 200MPa and a break elongation of 5% to 40% as measures at room temperature and at a speed of 5 mm/min.

## Patentansprüche

1. Polythioetherimid, wobei das Polythioetherimid eine Struktur der Formol I hat: 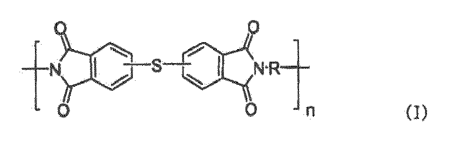 wobei die Thioetherbindung an der 3-Position oder 4-Position liegt;
wobei die organische Gruppe R ein substituiertes oder unsubstituiertes aliphatisches oder aromatisches Diamin ist; und
wobei das Polythioetherimid eine logarithmische Viskositätszahl von 0,13 dL/g bis 1,90 dL/g, gemessen in 0,5 g/dL m-Cresol bei 30°C, ein gewichtsgemitteltes Molekulargewicht von 3.000 bis 200.000 und eine Polydispersität von 1,8 bis 5,4 hat.

2. Polythioetherimid nach Anspruch 1, wobei die organische Gruppe R ausgewählt ist aus der Gruppe bestehend aus 1,6-Hexamethylendiamin, 1,6-cyclohexandiamin, p-Phenylendiamin, m-Phenylendiamin, 4,4'-Biphenylendiamin, 3,3'-Dimethyl-4,4'-biphenylendiamin, 2,2-Dimethyl-4,4'-biphenylendiamin, 4,4'-Diaminodiphenylether, 3,4'-Diaminodiphenylether, 4,4'-Diaminnobenzophenon, 3,4'-Diaminobenzophenon, 4,4'-Diaminodiphenylsulfon, 3,4'-Diaminodiphenylsulfon, 4,4'-Diaminodiphenylmethan, 4,4'-Diaminodiphenylisopropan, 4 ,4'-Diaminodiphenylthioether, 2,2'-Dichlor-4,4'-diaminodiphenylmethan, 3,3'-Dichlor-4,4'-diaminodiphenylmethan, 4,4'-Diaminodiphenoxyl-4",4"'-biphenyl, 4,4'-Diaminodiphenoxyl-4",4"'-diphenylether, 4,4'-Diaminodiphenoxyl-4",4"'-diphenylsulfon, 4,4'-Diaminodiphenoxyl-4",4"'-diphenylisopropan, 2,4-Toluoldiamin, 5-Methyl-4,6-diethyl-1,3-phenylendiamin, 3,3'-Dimethyl-4,4'-diaminodiphenylmethan oder 2,2',3,3'-Tetramethyl-4,4'-diaminodiphenylmethan oder Gemischen davon.

3. Verfahren zum Herstellen eines Polythioetherimids, wobei Chlorphthalsäureanhydrid- Nitrophthalsäureanhydridisomer der Formel II als Ausgangsmaterial zur Reaktion mit einem halbmolaren Äquivalent eines disubstituierten Amins NH₂RNH₂ verwendet wird, um ein disubstituiertes Phthalimid zu verhalten, das des Weiteren mit einem annähernd gleichmolaren Äquivalent eines Alkalimetallsulfids koppelt, um ein Polythioetherimidharz der Formel I, wie oben dargestellt, zu erhalten, 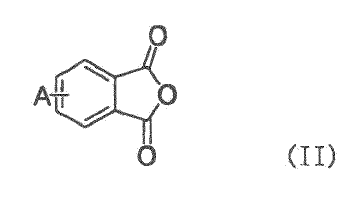 wobei Substitut A Chlor oder Nitro an der 3- oder 4-Position ist.

4. Herstellungsverfahren nach Anspruch 3, wobei das Molverhältnis von 3-substituiertem Phthalsäureanhydrid zu 4-substituiertem Phthalsäureanhydrid im Ausgangsmaterial, d.h. Chlorphthalsäureanhydrid oder Nitrophthalsäureanhydrid, in einem Bereich zwischen 99,9:0,1 und 0,1:99,9 liegt, oder wobei, das Herstellungsverfahren in zwei Schritten ausgeführt wird, wobei der erste Schritt die Reaktion zwischen dem Chlorophthalsäureanhydrid- oder Nitrophthalsäureanhydridisomer und einem halbmolaren Äquivalent eines disubstituierten Amins NH₂RNH₂ in einem polaren nicht-protonischen Lösemittel oder in Eisessigsäure unter Rückfluss oder in einem Gemisch aus einem benzolartigen Lösemittel, ausgewählt aus der Gruppe bestehend aus Benzol, Toluol, Xylol und Chlorbenzol, und einem polaren nicht photonischen Lösemittel unter Rückfluß bei einer Temperatur im Bereich von 100°C bis 200°C, besonders bevorzugt von 110°C bis 180°C beinhaltet; und der zweite Schritt die Kopplungsreaktion des erhaltenen disubstituierten Phthalimids mit einem gleichmolaren Äquivalent eines Alkalimetallsulfids in einem polaren nicht-protonischen Lösemittel oder in einem Gemisch aus einem benzolartigen Lösemittel, ausgewählt aus der Gruppe bestehend aus Benzol, Toluol, Xylol und Chlorbenzol, und einem polaren nicht photonischen Lösemittel in Gegenwart oder Abwesenheit eines optionalen Katalysators wie Natriumhydroxid, Kaliumhydroxid, wasserfreies Natriumcarbonat, wasserfreies Kaliumcarbonat oder wasserfreies Lithiumchlorid bei einer Temperatur im Bereich von 80°C bis 220°C, besonders bevorzugt von 100°C bis 170°C beinhaltet.

5. Herstellungsverfahren nach Anspruch 4, wobei das polare nicht-protonische Lösemittel N,N-Dimethylformamid (DMF), N,N-Dimethylacetamid (DMAc), N-Methyl-2-pyrrolidon (NMP), Dimethylsulfoxid (DMSO), Hexamethylphosphoramid (HMPA) oder Tetramethylensulfon ist oder wobei das benzolartige Lösemittel ausgewählt ist aus der Gruppe bestehend aus Benzol, Toluol, Xylol und Chlorbenzol, wobei das Alkalimetallsulfid wasserfreies Lithiumsulfid, Kalziumsulfid oder Natriumsulfid ist; und vorzugsweise das Alkalimetallsulfid durch eines folgenden Verfahren hergestellt wird:
(i) Reagieren eines Alkalimetalls mit Schwefel;
(ii) Reinigen eines Alkalimetallsulfids, insbesondere Natriumsulfid, industrieller Güte durch Erwärmen bei Hochvakuum oder durch azeotropischen Rückfluss mit einem benzolartigen Lösemittel, das ausgewählt ist aus der Gruppe bestehend aus Benzol, Toluol, Xylol und Chlorbenzol, Entfernung von Wasser, oder durch Rekristallisierung oder
wobei die organische Gruppe R ein substituiertes oder unsubstituiertes aliphatisches oder aromatisches Diamin ist.

6. Herstellungsverfahren nach Anspruch 5, wobei die organische Gruppe R ausgewählt ist aus der Gruppe bestehend aus 1,6-Hexamethylendiamin, 1,6-cyclohexandiamin, p-Phenylendiamin, m-Phenylendiamin, 4,4'-Biphenylendiamin, 3, 3'-Dimethyl-4,4'-biphenylendiamin, 2,2'-Dimethyl-4,4'-biphenylendiamin, 4,4'-Diaminodiphenylether, 3,4'-Diaminodiphenylether, 4,4'-Diaminobenzophenon, 3,4'-Diaminobenzophenon, 4,4'-Diaminodiphenylsulfon, 3,4'-Diaminodiphenylsulfon, 4,4'-Diaminodiphenylmethan, 4,4'-Diaminodiphenylisopropan, 4,4'-Diaminodiphenylthioether, 2 ,2'-Dichlor-4,4'-diaminodiphenylmethan, 3,3'-Dichlor-4,4'-diaminodiphenylmethan, 4 4'-Diaminodiphenoxyl-4",4"'-biphenyl, 4,4'-Diaminodiphenoxyl-4",4"'-diphenylether, 4,4'-Diaminodiphenoxyl-4",4"'-diphenylsulfon, 4,4'-Diaminodiphenoxyl-4",4"'-diphenylisopropan, 2,4-Toluoldiamin, 5-Methyl-4,6 -diethyl-1,3-phenylendiamin, 3,3'-Dimethyl-4,4'-diaminodiphenylmethan oder 2,2',3,3'-Tetramethyl-4,4'-diaminodiphenylmethan oder Gemischen davon.

7. Herstellungsverfahren nach Anspruch 4, wobei mindestens ein Ketten-Endkappenmittel zur Polymerisation verwendet wird, um den Polymerisationsgrad und das Molekulargewicht des endgültigen Polymers während Kopplungsreaktion disubstituierten Phthalimids mit dem Alkalimetallsulfid zu steuert.

8. Herstellungsverfahren nach Anspruch 7, wobei das Ketten-Endkappenmittel aromatische Verbindung der Formel III
B-Ar-M III
ist, wobei B ausgewählt ist aus Halogenatomen, wie Fluor, Chlor oder Brom, oder einer Nitrogruppe, ohne aber darauf beschränkt zu sein; Ar eine substituierte oder unsubstituierte aromatische Gruppe ist, die ausgewählt sein kann aus einem der Folgenden, ohne aber darauf beschränkt zu sein,: Phenyl, substituiertes Phenol, Biphenyl, substituiertes Biphenyl, Furanyl, Pyridyl, Naphthyl oder Chinolyl, usw.; M aus einem der folgenden Atome oder Gruppen ausgewählt sein kann, ohne aber darauf beschränkt zu sein: zum Beispiel Wasserstoff, Methyl, Acryl, Phenylacyl, Alkylsulphonyl, aromatisches Sulphonyl, Nitro, Cyano, Azo, Carboxyl, Trifluormethyl, Imide oder substituiertes Imido; und
vorzugsweise das Ketten-Endkappenmittel 3-Chlorphenyltert-butylketon, 3-Fluorophenyl-tert-butylketon, 4-Chlorbenzophenon, 3-Nitrobenzophenon, 4-Nitrophenylmethylsulfon, 4-Fluorphenylphenylsulfon, 2-Iodnitrobenzol, 4-Bromphenylazobenzol, 4-Fluorpyridin, 3-Chlorbenzoesäure, 1-Nitro-4-trifluormethylbenzol, 1-Chlor-3-trifluormethylbenzol, N-Phenyl-3-chlorphthalimid, N-Phenyl-4-fluorphthalimid, N-Methyl-3-chlorphthalimid, N-Methyl-4-nitrophthalimid, N-Butyl-3-chlorphthalimid oder N-Cyclohexyl-4-chlorphthalimid oder Gemische aus zwei oder mehr davon ist, wobei die bevorzugteste verwendete molare Menge des Ketten-Endkappenmittels das 0,01- bis 0,15-Fache jener des entsprechenden disubstituierten Phthalimids ist.

9. Verfahren zum Herstellen Polythioetherimids, wobei Chlorphthalsäureanhydrid- oder Nitrophthalsäureanhydridisomer der oben stehenden Formel II als Anusgangsmaterial zur Reaktion mit einem halbmolaren Äquivalenz eines organischen Diamins NH₂RNH₂ verwendet wird, um ein disubstituiertes Phthalimid zu erhalten, das des Weiteren mit einem annähernd gleichmolaren Äquivalent von Schwefel koppelt, um ein Polsthioetherimidharz der Formol I, wie dargestellt, zu erhalten.

10. Herstellungsverfahren nach Anspruch 9, wobei das Molverhältnis von 3-substituiertem Phthalsäureanhydrid zu 4-substituiertem Phthalsäureanhydrid im Ausgangsmaterial, d.h. Chlorphthalsäureanhydrid oder Nitrophthalsäureanhydrid, in einem Bereich zwischen 99,9:0,1 und 0,1:99,9 liegt, oder.
wobei das Herstellungsverfahren in zwei Schritten ausgeführt wird, wobei der erste Schritt die Reaktion zwischen isomerem Chlorophthalsäureanhydrid oder Nitrophthalsäureanhydrid und einem halbmolaren Äquivalent eines organischen Diamins in einem nichtphotonischen Lösemittel oder in Eisessigsäure unter Rückfluß oder in einem Gemisch aus einem benzolartigen Lösemittel, ausgewählt aus der Gruppe bestehend aus Benzol, Toluoyl, Xylol und Chlorbenzol, und einem polaren nicht photonischen Lösemittel unter Rückfluss oder in einem geschmolzenen Zustand unter Erwärmung, bei einer Temperatur im Bereich von 100°C, bis 350°C, bevorzugt von 120°C bis 280°C beinhaltet, um ein disubstituiertes Phthalimid zu erzeugen; und der zweite Schritt die Kopplungsreaktion des erhaltenen disubstituierten Phthalimids mit einem annähernd gleichmolaren Äquivalent von Schwefel in polaren nicht-protonischen Lösemittel oder in einem Gemisch aus einem benzolartigen Lösemittel, ausgewählt aus der Gruppe bestehend aus Benzol, Toluol, Xylol und Chlorbenzol, und einem polaren nicht photonischen Lösemittel in Gegenwart eines Reduktionsmittels, eines Katalysators und eines Reaktionshilfsmittels bei einer Temperatur im Bereich von 60°C bis 260°C, besonders bevorzugt von 100°C, bis 190°C beinhaltet, um Polythioetherimid zu erzeugen, wobei die verwendete molare Menge des Schwefels das 0,90- 90- bis 1,30-Fache, besonders bevorzugt das 0,95- bis 1,15-Fache jener des entsprechenden disubstituierten Phthalimids ist.

11. Herstellungsverfahren nach Anspruch 10, wobei das polare nicht-protonische Lösemittel N,N-Dimethylformamid (DMF), N,N-Dimethylacetamid (DMAc), N-Methyl-2-pyrrolidon (NMP), Dimethylsulfoxid (DMSO), Hexamethylphosphoramid (HMPA), Diphenylsulfon oder Tetramethylensulfon ist.

12. Herstellungsverfahren nach Anspruch 10, wobei das benzolartige Lösemittel ausgewählt ist aus der Gruppe bestehend Benzol, Toluoyl, Xylol und Chlorbenzol,
wobei die organische Gruppe R ein substituiertes oder unsubstituiertes aliphatisches oder aromatisches Diamin ist, das Beispiel aus mindestens einem der Folgenden ausgewählt sein kann, ohne aber darauf beschränkt zu sein: 1,2-Hexandiamin 1,6-Hexamethylendiamin, 1,6-Cyclohexandiamin, p-Phenylendiamin, m-Phenylendiamin, 4,4'-Biphenylendiamin, 3,3'-Dimethyl-4 4'-biphenylendiamin, 2,2'-Dimethyl-4,4'-biphenylendiamin, 4,4'-Diaminodiphenylether, 3,4'-Diaminodiphenylether, 4,4'-Diaminobenzophenon, 3,4'-Diaminobenzophenon, 4,4'-Diaminodiphenylsulfon, 3,4'-Diaminodiphenylsulfon, 4,4'-Diaminodiphenylmethan, 4,4'-Diaminodiphenylisopropan, 4,4'-Diaminodiphenylthioether, 2,2'-Dichlor-4,4'-diamin.dipienylmethan, 3,3'-Dichlor-4 ,4'-diaminodiphenylmethan, 4,4'-Diaminodiphenoxyl-4",4"'-biphenyl, 4,4'-Diaminodiphenoxyl-4",4"'-diphenylether, 4,4'-Diaminodiphenoxyl-4",4"'-diphenylsulfon, 4,4'-D.aminodiphenoxyl-4",4"'-diphenylisopropan, 2,4-Toluoldiamin, 5-Methyl-4,6--iethyl-1,3-phenylendiamin, 3,3'-Dimethyl-4,4'-diaminodiphenylmethan oder 2,2 ,3, '-Tetramethyl-4,4'-diaminodiphenylmethan oder Gemischen davon, oder
wobei das Reduktionsmittel für die Kopplung des disubstituierten Phthalimids mit Schwefel aus mindestens einem der Folgenden ausgewählt sein kann, ohne aber darauf beschränkt zu sein: Formate (wie Natriumformate, Kaliumformat oder Lithiumformat), Oxalate (wie Natriumoxalat, Kaliumoxalat oder Lithiumoxalat), Aldehyde (wie Formaldehyd oder Acetaldehyd), Hydrazine (wie Phenylhydrazin oder hydriertes Hydrazin), Hydroxylamin, elementares Metall (wie Eisenpulver, Aluminiumpulver oder Zinkpulver), Hydride (wie Natriumhydrid, Kaliumhydrid, Natriumborhydrid oder Lithiumaluminiumhydrid), Ammoniak, Wasserstoff und dergleichen oder Gemischen davon; und die verwendete molare Menge des Reduktionsmittels das 0,2- bis 6-Fache, besonders bevorzugt das 0,4- bis 3-Fache jener von Schwefel ist, oder wobei das Hilfsmittel und der Katalysator für die Kopplungsreaktion des disubstituierten Phthalimids mit Schwefel aus mindestens einem der Folgenden ausgewählt sein können, ohne aber darauf beschränkt zu sein: Carbonate wie Lithiumcarbonat, Natriumcarbonat oder Kaliumcarbonat, Kohlenwasserstoffe, wie Natriumhydrogencarbonat oder Kaliumhydrogencarbonat, Phosphate wie Natriumhydrophosphat oder Kaliumhydrophosphat, Hydrophosphate wie dibasisches Natriumphosphat oder disbasisches Kaliumphosphat, basische Hydroxide wie Kaliumhydroxid, Natriumhydroxid oder Lithiumhydroxid, Halogenide, wie Calziumchlorid, Natriumchlorid, Kaliumchlorid, Lithiumbromid, Kalziumfluorid oder Natriumiodid, oder Gemische davon; und
die verwendete molare Menge des Hilfsmittels oder Katalysators das 0,02- bis 3-Fache, besonders bevorzugt das 0,05- bis 1,5-Fache jener von Schwefel ist, oder
wobei die Kopplungspolymerisation disubstituierten Phthalimids mit Schwefel in einer inerten Atmosphäre ausgeführt wird, die aus Stickstoff oder Argon ausgewählt werden kann, ohne aber darauf beschränkt zu sein.

13. Herstellungsverfahren nach Anspruch 10, wobei mindestens ein Ketten-Endkappenmittel zur Polymerisation verwendet wird, um den Polymerisationsgrad und das Molekulargewicht des endgültigen Polymers während der Kopplungsreaktion des disubstituierten Phthalimids mit dem Schwefel zu steuern.

14. Herstellungsverfahren nach Anspruch 13, wobei das Ketten-Endkappenmittel eine aromatische Verbindung der Formel III
B-Ar-M III
ist, wobei B ausgewählt sein kann aus Halogenatomen, wie Fluor, Chlor oder Brom, oder einer Nitro, usw ohne aber darauf beschränkt zu sein; Ar eine substituierte oder unsubstituierte aromatische Gruppe ist, die ausgewählt sein kann aus einem der Folgenden, ohne aber darauf beschränkt zu sein,: Phenyl, substituiertes Phenyl, Biphenyl, substituiertes Biphenyl, Furanyl, Pyridyl, Naphthyl oder Chinolyl, usw.; M aus einem der folgenden Atome oder Gruppen ausgewählt sein kann, ohne aber darauf beschränkt zu sein: zum Beispiel Wasserstoff, Methyl, Acyl, Phenylacyl, Alkylsulphonyl, aromatisches Sulphonyl, Nitro, Cyano, Azo, Carboxyl, Trifluormethyl, Imido oder substituiertes Imido; und vorzugsweise das Ketten-Endkappenmittel 3-Chlorphenyltert-butylketon, 3-Fluorophenyl-tert-butylketon, 4-Chlorbenzophenon, 3-Nitrobenzophenon, 4-Nitrophenylmethylsulfon, 4-Fluorphenylphenylsulfon, 2-Iodnitrobenzol, 4-Bromphenylazobenzol, 4-Fluorpyridin, 3-Chlorbenzoesäure, 1-Nitro-4-trifluormethylbenzol, 1-Chlor-3-trifluormethylbenzol, N-Phenyl-3-chlorphthalimid, N-Phenyl-4-fluorphthalimid, N-Methyl-3-chlorphthalimid, N-Methyl-4-nitrophthalimid, N-Butyl-3-chlorphthalimid oder N-Cyclohexyl-4-chlorphthalimid oder Gemische aus zwei oder mehr davon ist, wobei die bevorzugteste verwendete molare Menge des Ketten-Endkappenmittels das 0,01- bis 0,15-flache jener des entsprechenden disubstituierten Phthalimids ist.

15. Herstellungsverfahren nach einem der Ansprüche 3 bis 14, wobei das Polythioetherimid eine logarithmische Viskositätszahl von 0,13 dL/g bis 1,90 dL/g, gemessen in 0,5 g/dL m-Cresol bei 30°C hat, oder wobei das Polythioetherimid ein gewichtsgemitteltes Molekulargewicht vorn 3.000 bis 200.000 und eine Polydispersität von 1,8 bis 5,4 hat, oder wobei das Polythioetherimid eine Glasübergangstemperatur von 200°C bis 350°C laut Wiedererwärmungsdaten hat, oder
wobei das Polythioetherimid eine Viskosität von 500P bis 100.000P, gemessen bei einer Temperatur von 380°C und bei einer Geschwindigkeit von 1000 s⁻¹ hat, oder
wobei das Polythioetherimid eine Filmzugfestigkeit von 60MPa bis 200 und eine Bruchdehnung von 5% bis 40% gemessen bei Raumtemperatur und bei einer Geschwindigkeit von 5 mm/min hat.

## Revendications

1. Un polythioetherimide, dans lequel le polythioetherimide a une structure de formule I: 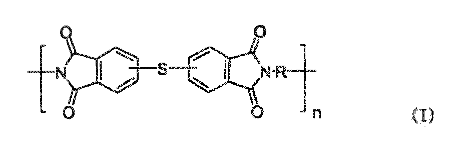 dans laquelle la liaison thioéther est située à la position 3 ou à la position 4;
dans laquelle le groupe organique est une diamine aliphatique ou aromatique substituée ou non substituée; et
dans lequel ledit polythioetherimide a un indice de viscosité logarithmique de 0,13 dL/g à 1,90 dL/g mesuré dans 0,5 g/dL de m-crésol à 30°C, un poids moléculaire moyen en poids de 3.000 à 200.000 et une polydispersité de 1,8 à 5,4.

2. Le polythioetherimide de la revendication 1, dans lequel le groupe organique est choisi parmi le groupe constitué par le 1,6-hexanléthylène-diamine, la 1,6-cyclohexanediamine, le p-phénylène diamine, la m-phényléne diamine, le 4,4'-biphényléne-diamine, la 3,3'-diméthyl-4, 4'-biphénylène-diamine, la 2,2 '-diméthyl-4, 4'-biphénylène-diamine, le 4,4'-diaminodiphényl éther, le 3,4'-diaminodiphényléther, la 4,4'-diaminobenzophénone, la 3,4'-diaminobenzophénone, la 4,4'-diaminodiphényl-sulfone, la 3,4'-diaminodiphénylsulfone, le 4,4'-diaminodiphénylméthane, le 4,4'-diaminodiphényl isopropane, le 4,4'-diaminodiphényl thioéther, le 2,2 '-dichloro-4,4'-diaminodiphénylméthane, le 3,3'-dïchloro-4,4'-diaminodiphénylméthane, le 4,4' -diaminodiphenoxyl-4",4"'-biphényle, le 4,4"-diaminodiphenoxyl-4",4"'-diphényléther, la 4,4'-diamino-diphenoxy-4",4"'-diphénylsulfone, le 4,4'-diaminodiphenoxy-4",4"'-diphényl isopropane, la 2,4-toluène diamine, la 5-méthyl-4,6-diéthyl-1,3-phénylène diamine, le 3,3'-diméthyl-4,4'-dïaminodiphényl méthane, ou le 2,2',3,3'-tétraméthyl-4,4'-diaminodiphénylméthane, ou leurs mélanges.

3. Procédé de préparation d'un polythioetherimide, dans lequel un isomère de l'anhydride chlorophtalique ou de l'anhydride nitrophtalique de formule Il est utilisé en tant que le matériau de départ pour réagir avec la moitié d'un équivalent molaire d'une aminé disubstituée NH₂RNH₂ pour donner un imide phthalique disubstitué qui se couple en outre avec environ un équivalent molaire d'un sulfure de métal alcalin pour donner une résine de polythioetherimide de formule 1 comme montrée ci-dessus, 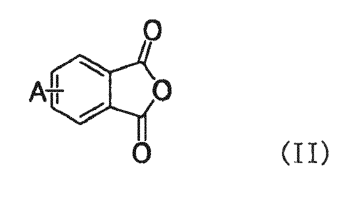 dans laquelle le substituant A est le chlore ou nitro à la position 3 ou 4.

4. Le procédé de préparation de la revendication 3, dans lequel le rapport molaire de l'anhydride phtalique substitué en 3 à l'anhydride phtalique substitué en 4 dans le produit de départ, à savoir l'anhydride chlorophtalique ou l'anhydride nitrophtalique, est dans la gamme comprise entre 99,9 : 0,1 et 0,1 : 99,9, ou dans lequel le procédé de préparation est is en oeuvre en deux étapes, dans lequel la première étape comprend la réaction entre l'isomère d'anhydride chlorophtalique ou d'anhydride nitrophtalique et la moitié d'un équivalent molaire d'une amine disubstituée NH₂RNH₂ dans un solvant polaire non protoné, ou dans de l'acide acétique glacial au reflux, ou dans un mélange d'un solvant de type benzène choisi parmi le groupe constitué du benzène, du toluène, du xylène et du chlorobenzène et d'un solvant polaire non protoné au reflux, à une température allant de 100 °C à 200 °C, de préférence de 110 °C à 180 °C, et dans lequel la seconde étape comprend la réaction de couplage de l'imide phtalique disubstitué résultant avec un équivalent molaire d'un sulfure de métal alcalin dans un solvant polaire non protoné, ou dans mélange d'un solvant de type benzène choisi parmi le groupe constitué du benzène, du toluène, du xylène et du chlorobenzène et d'un solvant polaire non protoné, en présence ou en l'absence d'un catalyseur facultatif tel que l'hydroxyde de sodium, l'hydroxyde de potassium, le carbonate de sodium anhydre, le carbonate de potassium anhydre ou le chlorure de lithium anhydre, à une température allant de 80 °C à 220 °C, de préférence de 100 °C à 170 °C.

5. Le procédé de préparation de la revendication 4, dans lequel le solvant polaire non protoné est le N,N'-diméthyl-formamide (DMF), le N, N'-diméthylacétamide (DMAc), la N-méthyl-2-pyrrolidone (NMP), le diméthylsulfoxyde (DMSO), l'hexaméthyl-phosphoramide (HMPA) ou la tétraméthylénesulfone, ou dans lequel le solvant de type benzène est choisi parmi le groupe constitué du benzène, du toluène, du xylène et du chlorobenzène, dans lequel le sulfure de métal alcalin est le sulfure de lithium anhydre, le sulfure de potassium ou le sulfure de sodium, et de préférence, le sulfure de métal alcalin est préparé par l'intermédiaire d'une des deux méthodes suivantes:
(I) la réaction d'un métal alcalin avec du soufre;
(Ii) la purification d'un sulfure de métal alcalin de qualité industrielle, en particulier le sulfure de sodium, par chauffage sous vide poussé, ou par reflux azéotropique avec un solvant de type benzène choisi parmi le groupe constitué benzène, du toluène, du xylène et du chlorobenzène afin d'éliminer l'eau, ou par recristallisation, ou
dans lequel le groupe organique est une diamine aliphatique ou aromatique substituée ou non substituée.

6. Le procédé de préparation conforme à la revendication 5, dans lequel le groupe organique est choisi parmi le groupe constitué par la 1,6-hexaméthylène-diamine, la 1,6-cyclohexanediamine, le p-phénylène diamine, la m-phényléne diamine, le 4,4'-biphénylène-diamine, la 3,3'-diméthyl-4,4'-biphénylène diamine, la 2,2'-diméthyl-4, 4'-biphénylène-diamine, le 4,4'-diaminodiphényléther, le 3,4'-diaminodiphényléther, la 4,4'-diaminobenzophénone, la 3,4'-diaminobenzophénone, la 4,4'-diaminodiphényl sulfone, la 3,4'-diaminodiphényl sulfone, la 4,4'-diaminodiphénylméthane, le 4,4'-diaminodiphényl isopropane, le 4,4'-diaminodiphényl thioéther, le 2,2'-dichloro-4,4'-diaminodiphénylméthane, le 3,3'-dichloro-4,4'-diaminodiphénylméthane, le 4.4'-diaminodiphenoxy-4",4"'-biphényle, l'éther de 4,4'-diaminodiphénoxy-4",4"'-diphényle, la 4,4'-diaminodiphenoxy-4",4"'-diphénylsulfone, le 4,4'-diaminodiphenoxy-4",4"'-diphényl isopropane, la 2,4-toluène diamine, la 5-methyl-4,6-diéthyl-1,3-phényléne diamine, le 3,3'-diéthyl-4,4'-diaminodiphényl méthane, ou le 2,2',3,3'-tétraméthyl-4, 4'-diaminodiphénylméthane, ou leurs mélanges.

7. Le procédé de préparation de la revendication 4, dans lequel on utilise au moins un agent de terminaison de chaîne pour la polymérisation afin de contrôler le degré de polymérisation et le poids moléculaire du polymère final au cours de la réaction de couplage de l'imide phtalique disubstitué avec le sulfure de métal alcalin.

8. Le procédé de préparation de la revendication 7, dans lequel l'agent de terminaison de chaîne est un composé aromatique de formule III,
B-Ar-M (III)
dans laquelle est choisi parmi, mais sans s'y limiter, des atomes d'halogène tels que le fluor, le chlore ou le brome, ou un groupe nitro; Ar est un groupe aromatique substitué ou non qui peut être choisi parmi, mais sans s'y limiter, l'un des suivants: phényle, phényle substitué, biphényle, biphényle substitué, furanyle, pyridyle, naphthyle ou quinolyle, etc.; peut être choisi à partir de, mais sans s'y limiter, l'un des atomes ou groupes suivants: par exemple, hydrogène, éthyle, acyle, phénylacyle, alcoylsulfonyle, sulfonyle aromatique, nitro, cyano, azo, carboxyle, triftuorométhyle, imido ou imido substitué, et
de préférence, l'agent de terminaison de chaîne est la 3-chlorophényl-tert-butyl cétone, la 3-fluorophényl-tert-butyl cétone, la 4-chlorobenzophénone, la 3-nitrobenzophénone, la 4-nitrophényl méthylsulfone, la 4-fluorophényl phénylsulfone, le 2-iodonitrobenzene , le 4-bromophényl azobenzène, la 4-fluoropyridine, l'acide 3-chlarabenzoïque, le 1-nitro-4-trifluorométhylbenzène, le 1-chloro-3-trifluorométhyl-benzène, N-phényl-3-chlorophtalique imide, le N-phényl-4-fluorophtalique imide imide, le N-méthyl-3-chlorophthalique imide, le N-méthyl-4-nitrophthalique imide, le N-butyl-3-chlorophthalique imide, ou le N-cyclohexyl-4-chlorophthalique imide, ou des mélanges de deux ou plusieurs de ceux-ci, dans lequel la quantité molaire d'agent de terminaison de chaîne utilisé la plus préférée est environ 0,01 à 0.15 fois celle de l'imide phthalique disubstitué correspondant.

9. Un procédé de préparation d'un polythioetherimide, dans lequel de l'anhydride chlorophthalique ou de l'anhydride nitrophthalique de la formule II ci-dessus est utilisé en tant que matériau de départ pour réagir avec une moitié d'équivalent molaire d'une diamine organique NH₂RNH₂ pour donner un imide phthalique disubstitué qui se couple en outre avec environ un équivalent molaire de soufre pour donner une résine de polythioetherimide de la formule 1 comme montrée ci-dessus.

10. Le procédé de préparation de la revendication 9, dans lequel le rapport molaire de l'anhydride phthalique 3-substitué à l'anhydride phthalique 4-substitué dans le produit de départ, à savoir l'anhydride chlorophthalique ou l'anhydride nitrophthalique est dans n'importe quelle gamme comprise entre 99,9 : 0,1 et 0,1 : 99,9, ou dans lequel le procédé de préparation est mis en oeuvre en deux étapes, dans lequel la première étape comprend la réaction entre l'isomère d'anhydride chlorophthalique ou d'anhydride nitrophthalique et la moitié d'un équivalent molaire d'une diamine organique dans un solvant polaire non protoné, ou dans de l'acide acétique glacial au reflux, ou dans un mélange d'un solvant de type benzène choisi parmi le groupe constitué du benzène, du toluène, du xylène et du chlorobenzéne et d'un solvant polaire non protoné au reflux, ou à l'état fondu sous chauffage, à une température allant de 100 °C à 350 °C, de préférence de 120 °C à 280 °C, pour produire un imide phthalique disubstitué, et la seconde étape comprend la réaction de couplage de l'imide phthalique disubstitué avec environ un équivalent molaire de soufre dans un solvant polaire non protoné ou dans un mélange d'un solvant de type benzène choisi parmi le groupe constitué du benzène, du toluène, du xylène et du chlorobenzène et d'un solvant polaire non protoné en présence d'un agent réducteur, un catalyseur et un agent auxiliaire de réaction, à une température allant de 60 °C à 260 °C, de préférence de 100 °C à 190 °C, pour produire du polythioetherimide, dans lequel la quantité molaire de soufre utilisé est environ de 0,90 à 1.30 fois, plus préférablement 0,95 à 1,1 fois celle de l'imide phthalique disubstitué correspondant.

11. Le procédé de préparation de la revendication 10, dans lequel le solvant polaire non protoné est le N,N-diméthylformamide (DMF), le N,N-diméthylacétamide (DMAc), la N-méthyl-2-pyrrolidone (NMP), le diméthylsulfoxyde (DMSO), l'hexméthyl-phosphoramide (HMPA), la diphénylsulfone ou la têtraméthylènesulfone.

12. Le procédé de préparation de la revendication 10, dans lequel le solvant de type benzène est choisi parmi le benzène, le toluène, le xylène et le chlorobenzène, dans lequel le groupe organique est une diamine aromatique ou aliphatique substituée ou non substituée qui peut être choisie parmi, mais sans s'y limiter, par exemple, au moins un des éléments suivants: la 1,2-hexanediamine, la 1,6-hexaméthylène-diamine, la 1,6-cyclohexanediamine, la p-phénylénediamine, la m-phénylène diamine, le 4,4 '-biphényléne-diamine, la 3,3'-diméthyl-4,4'-biphénylène-diamine, la 2,2'-diméthyl- le 4,4'-biphényléne-diamine, le 4,4'-diaminodiphényl éther, le 3,4'-diaminodiphényl éther, la 4,4'-diaminobenzophénone, la 3,4'-diaminobenzophénone, la 4,4'-diaminodiphénylsulfone, la 3,4'-diaminodiphénylsulfone, la 4,4'-diaminodiphénylméthane, le 4,4'-diaminodiphenyl isopropane, le 4,4'-diaminodiphényl thioéther, le 2,2'-dichloro-4,4'-diaminodiphénylméthane, le 3,3'-dichloro-4,4'-diaminodiphénylméthane, le 4,4'-diaminodiphenoxyl-4",4"'-biphényle, le 4,4'-diaminodiphénoxy-4",4"'-diphényl éther, la 4,4'-diaminodiphenoxy-4",4"'-diphényl." sulfone, le 4,4'-diaminodiphenoxyl-4",4"'-dïphényl isopropane, la 2,4-toluéne diamine, la 5-méthyl-4,6-diéthyl-1,3-phényléne diamine, le 3,3'-diméthyl-4,4'-diaminodiphénylméthane ou le 2,2',3,3'-tétraméthyl-4,4'-diaminodiphénylméthane ou leurs mélanges, ou
dans lequel l'agent réducteur pour le couplage de l'imide phthalique disubstitué avec du soufre peut être choisi parmi, mais sans s'y limiter, au moins un des éléments suivants: des formiates (tels que le formiate de sodium, le formiate de potassium ou le formiate de lithium), des oxalates (tels que l'oxalate de sodium, l'oxalate de potassium ou l'oxalate de lithium), des aldéhydes (comme le formaldéhyde ou l'acétaldéhyde), des hydrazines (comme la phényl-hydrazine ou de l'hydrazine hydratée), l'hydroxylamine, du métal élémentaire (comme de la poudre de fer, de la poudre d'aluminium ou de la poudre de zinc), des hydrures (tels que l'hydrure de sodium, l'hydrure de calcium, le borohydrure de sodium ou l'hydrure de lithium et d'aluminium), l'ammoniac, l'hydrogène et ainsi de suite, ou leurs mélanges, et la quantité molaire de l'agent réducteur utilisé est de 0,2 à 6 fois, de préférence de 0,4 à 3 fois, celle du soufre, ou
dans lequel l'auxiliaire et le catalyseur pour la réaction de couplage de l'imide phthalique disubstitué avec du soufre peut être choisi parmi, mais sans s'y limiter, au moins une des suivants: des carbonates tels que le carbonate de lithium, le carbonate de sodium ou le carbonate de potassium, des hydrocarbonates tels que l'hydrocarbonate de sodium ou l'hydrocarbonate de potassium, des phosphates tels que l'hydrophosphate de sodium ou l'hydrophosphate de potassium, des hydrophosphates tels que le phosphate de sodium dibasique ou le phosphate de potassium dibasique, des hydroxydes basiques comme l'hydroxyde de potassium, l'hydroxyde de sodium ou l'hydroxyde de lithium, des halogénures tels que le chlorure de calcium, le chlorure de sodium , le chlorure de potassium, le bromure de lithium, le fluorure de potassium ou l'iodure de sodium, ou des mélanges de ceux-ci, et
la quantité molaire de l'auxiliaire et du catalyseur utilisé est de 0,02 à 3 fois, de préférence de 0,05 à 1,5 fois celle du soufre, ou
dans lequel la polymérisation par couplage de l'imide phthalique disubstitué avec du soufre est effectuée dans une atmosphère inerte qui peut être choisi parmi, mais sans s'y limiter, l'azote ou l'argon.

13. Le procédé de préparation de la revendication 10, dans lequel on utilise au moins un agent de terminaison de chaîne pour la polymérisation pour contrôler le degré de polymérisation et le poids moléculaire du polymère final au cours de la réaction de couplage de l'imide phthalique disubstitué et du soufre.

14. Le procédé de préparation de la revendication 13, dans lequel l'agent de terminaison de chaîne est un composé aromatique de formule III,
B-Ar-M (III)
où peut être choisi parmi, mais sans s'y limiter, les atomes d'halogène tels que le fluor, le chlore ou le brome, ou un groupe nitro, etc; Ar est un groupe aromatique substitué ou non qui peut être choisi parmi, mais sans s'y limiter, l'un des suivants: phényle, phényle substitué, biphényle, biphényle substitué, furanyle, pyridyle, naphtyle ou quinolyle; peut être choisi parmi, mais sans s'y limiter, l'un des atomes ou groupes suivants: par exemple, un atome hydrogène, méthyle, acyle, phénylacyle, alcoylsulfonyle, sulfonyle aromatique, nitro, cyano, azo, carboxyle, trifluorométhyle, imido ou imido substitué, et
de préférence, l'agent de terminaison de chaîne est la 3-chlorophényl-tert-butyl cétone, la 3-fluorophényl-tert-butyl cétone, la 4-chlorobenzophénone, la 3-nitrobenzophénone, la 4-nitrophényl méthyl sulfone, la 4-fluorophényl phénylsulfone, le 2-iodonitrobenzène, le 4-bromophényl azobenzène, la 4-fluoropyridine, l'acide 3-chlorobenzoïque, le 1-nitro-4-trifluorométhyl benzène, le 1-chloro-3-trifluorométhyl benzène, le N-phényl-3-chlorophthalimide, le N-phényl-4-fluorophtalimide , le N-méthyl-3-chlorophtalimide, le N-méthyl-4-nitrophtalimide, le N-butyl-3-chlorophtalimide, ou le N- cyclohexyl-4-chlorophtalimide, etc, ou des mélanges de deux ou plusieurs de ceux-ci, dans lequel la quantité molaire préféré de l'agent de terminaison de chaîne utilisé est de 0,01 à 0,15 fois celle de l'imide phtalique disubstitué correspondant.

15. Le procédé de préparation selon l'une quelconque des revendications 3 à 14, dans lequel le polythioetherimide a un indice de viscosité logarithmique de 0,13 dL/g à 1,90 dL/g tel que mesuré dans 0,5 g/dL de m-crésol à 30 °C, ou dans lequel le polythioetherimide a un poids moléculaire moyen en poids de 3.000 à 200.000 et une polydispersité de 1,8 à 5,4, ou dans lequel le polythioetherimide a une température de transition vitreuse de 200 °C à 350 °C conformément aux données de recuit, ou
dans lequel le polythioetherimide a une viscosité de 500 P à 100.000 P, telle que mesurée à une température de 380 °C et à une vitesse de 1000 s⁻, ou
dans lequel le polythioetherimide a une résistance à la traction du film de 60 MPa à 200 MPa et un allongement à la rupture de 5 % à 40 %, telle que mesu-rée à température ambiante et à une vitesse de 5 mm / min.
